# EUROPEAN PATENT APPLICATION

(11) **EP 4 521 033 A1**
(43) Date of publication of application: **12.03.2025**
(21) Application number: 24171054.0
(22) Date of filing: 18.04.2024
(51) Int. Cl.: F24F 7/007, F24F 8/108, F24F 11/00, F24F 11/58, F24F 7/003

(54) **INDOOR AIR CLEANING SYSTEM**

(30) Priority: 06.09.2023 TW 112133943
(71) Applicant: Microjet Technology Co., Ltd., Hsinchu (TW)
(72) Inventor: Mou, Hao-Jan, Hsinchu (TW); Wu, Chin-Chuan, Hsinchu (TW); Huang, Chi-Feng, Hsinchu (TW)
(74) Representative: Uexküll & Stolberg

(57) **Abstract**

An indoor air cleaning system includes plural gas detectors (1) disposed in outdoor field (A) and indoor field (B) for detecting air pollution information and outputting thereof via IOT communication; at least one cleaning device (2) including a fan (21), a filter element (22) and a sterilization component (23), wherein the fan (21) guides an air pollution to pass through the filter element (22) and the sterilization component (23), the fan (21) has a specific CADR for generating a directional circular airflow, and the filter element (22) includes a HEPA filter screen; and a cloud computing server (4) receiving the indoor and outdoor air pollution information via IOT communication, storing the air pollution information to an air pollution big data database, and performing intelligent computing to output a control command to the cleaning device (2) to generate the directional circular airflow for rapidly guiding the air pollution to pass through the filter element (22) and the sterilization component (23), thereby reaching a gas state of the indoor field (B) cleanroom classes.

## Description

### FIELD OF THE INVENTION

The present disclosure relates to an indoor air cleaning system, and more particularly to an indoor air cleaning system for reaching the gas state of an indoor field to the cleanroom class by a cleanliness specification based on a passage number of particles.

### BACKGROUND OF THE INVENTION

Suspended particles are solid particles or droplets contained in the air. Due to their extremely fine size, the suspended particles may enter the lungs of human body through the nasal hairs in the nasal cavity easily, causing inflammation in the lungs, asthma or cardiovascular disease. If other pollutant compounds are attached to the suspended particles, it will further increase the harm to the respiratory system. In recent years, the problem of air pollution is getting worse. In particular, the concentration of particle matters (e.g., PM_{2.5}) is often too high. Therefore, the monitoring to the concentration of the gas suspended particles is taken more and more seriously. However, the gas flows unstably due to variable wind direction and air volume, and the general gas-quality monitoring station is located in a fixed place. Under this circumstance, it is impossible for people to check the concentration of suspended particles in current environment.

Furthermore, in recent years, modern people are placing increasing importance on the quality of the air in their surroundings. For example, carbon monoxide, carbon dioxide, volatile organic compounds (VOC), PM_{2.5}, nitric oxide, sulfur monoxide and even the suspended particles contained in the air are exposed in the environment to affect the human health, and even endanger the life seriously. Therefore, the quality of environmental air has attracted the attention of various countries. At present, how to detect the air quality and avoid the harm is a crucial issue that urgently needs to be solved.

In order to confirm the quality of the air, it is feasible to use a gas sensor to detect the air in the surrounding environment. If the detection information can be provided in real time to warn people in the environment, it is helpful of avoiding the harm and facilitates people to escape the hazard immediately, preventing the hazardous gas exposed in the environment from affecting the human health and causing the harm. Therefore, it is considered a valuable application to use a gas sensor to detect the air in the surrounding environment.

In addition, it is not easy to control the indoor air quality. Besides the outdoor air quality, indoor air-conditioning conditions and pollution sources are the major factors affecting the indoor air quality. It is necessary to intelligently and quickly detect indoor air pollution sources in various indoor fields, effectively remove the indoor air pollution to form a clean and safe breathing gas state, and monitor the indoor air quality in real time anytime, anywhere. Certainly, if the concentration of the suspended particles in the indoor field is strictly controlled according to the "clean room" standard, it allows avoiding the introduction, generation and retention of suspended particles, and the temperature and humidity in the indoor field can be controlled within the required range. That is to say, through classifying the indoor field by the number of suspended particles in the air and the number of suspended particles per cubic meter in the air pollution, the indoor field can meet the clean room requirements for safe breathing. Therefore, it is a main subject developed in the present disclosure to provide a solution of detecting the indoor air quality and solve the problem of air pollution, so that the indoor field can meet the clean room requirements, and the impact and injury for human health caused by the gas hazards in the environment can be avoided.

### SUMMARY OF THE INVENTION

The major object of the present disclosure is to provide an indoor air cleaning system. By disposing a plurality of gas detectors and at least one cleaning device in the outdoor and indoor fields, the gas detectors can monitor and determine air pollution information at any time. Then, a cloud computing server receives the air pollution information via Internet of Things (IOT) communication, stores the air pollution information to an air pollution big data database, performs the intelligent computing to compare and determine the location of the air pollution, and intelligently issues a control command via IOT communication to a fan of the cleaning device for regulating the enablement and the air volume, so that a directional circular airflow is generated in the indoor field, and the air pollution is rapidly guided to pass through the filter element multiple times for filtering the air pollution to a level close to zero and pass through the sterilization component for sterilization, thereby improving the purification efficiency and reducing the environmental noises in the indoor field. Such indoor air cleaning system forms a detecting-cleaning prevention effectiveness of locating the air pollution, guiding the air pollution, and purifying the air pollution to a level close to zero. Moreover, the filter element of the cleaning device employs the HEPA filter screen to filter the air pollution, and the fan of the cleaning device employs a specific CADR (Clean Air Delivery Rate) to generate the directional circular airflow, so that the gas state of the indoor field can reach the cleanroom class by a cleanliness specification based on the passage number of particles, and the impact and injury for human health caused by the gas hazards in the environment can be avoided.

In a broader aspect of the present disclosure, an indoor air cleaning system is provided. The indoor air cleaning system includes a plurality of gas detectors disposed in an outdoor field and an indoor field for detecting air pollution information and outputting thereof via Internet of Things (IOT) communication; at least one cleaning device including a fan, a filter element and a sterilization component, wherein the fan is enabled and controlled via IOT communication to guide an air pollution to pass through the filter element for filtration, and pass through the sterilization component for sterilization, and wherein the fan has a specific clean air delivery rate (CADR) for generating a directional circular airflow, and the filter element comprises a high efficiency particulate air (HEPA) filter screen; and a cloud computing server receiving the air pollution information of the outdoor field and the indoor field via IOT communication, storing the air pollution information to an air pollution big data database, performing an intelligent computing for comparison based on the big data database, and intelligently selecting and issuing a control command via IOT communication to enable the fan of the at least one cleaning device to generate the directional circular airflow for rapidly guiding the air pollution to pass through the filter element multiple times for filtration to a level close to zero and pass through the sterilization component for sterilization, thereby reaching a gas state in the indoor field to a cleanroom class by a cleanliness specification based on a passage number of particles.

### BRIEF DESCRIPTION OF THE DRAWINGS

The above contents of the present disclosure will become more readily apparent to those ordinarily skilled in the art after reviewing the following detailed description and accompanying drawings, in which:
FIG. 1 is a schematic view illustrating an indoor air cleaning system implemented in an indoor field according to an embodiment of the present disclosure;
FIG. 2A is a schematic view illustrating the combination of a fan and a filter element of a cleaning device according to the embodiment of the present disclosure;
FIG. 2B is a schematic view illustrating the combination of the filter element and a sterilization component of the cleaning device according to the embodiment of the present disclosure;
FIG. 3A is a schematic perspective view illustrating a gas detector according to the embodiment of the present disclosure;
FIG. 3B is a schematic perspective view illustrating the gas detector according to the embodiment of the present disclosure and taken from another perspective;
FIG. 3C is a schematic perspective view illustrating a gas detection module installed inside the gas detector according to the embodiment of the present disclosure;
FIG. 4A is a schematic perspective view (1) illustrating a gas detection main part according to the embodiment of the present disclosure;
FIG. 4B is a schematic perspective view (2) illustrating the gas detection main part according to the embodiment of the present disclosure;
FIG. 4C is an exploded view illustrating the gas detection main part according to the embodiment of the present disclosure;
FIG. 5A is a schematic perspective view (1) illustrating a base according to the embodiment of the present disclosure;
FIG. 5B is a schematic perspective view (2) illustrating the base according to the embodiment of the present disclosure;
FIG. 6 is a schematic view (3) illustrating the base according to the embodiment of the present disclosure;
FIG. 7A is a schematic exploded view illustrating the combination of a piezoelectric actuator and the base according to the embodiment of the present disclosure;
FIG. 7B is a schematic perspective view illustrating the combination of the piezoelectric actuator and the base according to the embodiment of the present disclosure;
FIG. 8A is a schematic exploded view (1) illustrating the piezoelectric actuator according to the embodiment of the present disclosure;
FIG. 8B is a schematic exploded view (2) illustrating the piezoelectric actuator according to the embodiment of the present disclosure;
FIG. 9A is a schematic cross-sectional view (1) illustrating an action of the piezoelectric actuator according to the embodiment of the present disclosure;
FIG. 9B is a schematic cross-sectional view (2) illustrating an action of the piezoelectric actuator according to the embodiment of the present disclosure;
FIG. 9C is a schematic cross-sectional view (3) illustrating an action of the piezoelectric actuator according to the embodiment of the present disclosure;
FIG. 10A is a schematic cross-sectional view (1) illustrating the gas detection main part according to the embodiment of the present disclosure;
FIG. 10B is a schematic cross-sectional view (2) illustrating the gas detection main part according to the embodiment of the present disclosure;
FIG. 10C is a schematic cross-sectional view (3) illustrating the gas detection main part according to the embodiment of the present disclosure;
FIG. 11 is a block diagram illustrating the communication of the gas detector according to the embodiment of the present disclosure.
FIG. 12 is a block diagram showing the architecture of a cloud computing server according to the embodiment of the present disclosure; and
FIG. 13 is a diagram showing the classifications of clean room according to the embodiment of the present disclosure.

### DETAILED DESCRIPTION OF THE PREFERRED EMBODIMENT

The present disclosure will now be described more specifically with reference to the following embodiments. It is to be noted that the following descriptions of preferred embodiments of this disclosure are presented herein for purpose of illustration and description only. It is not intended to be exhaustive or to be limited to the precise form disclosed.

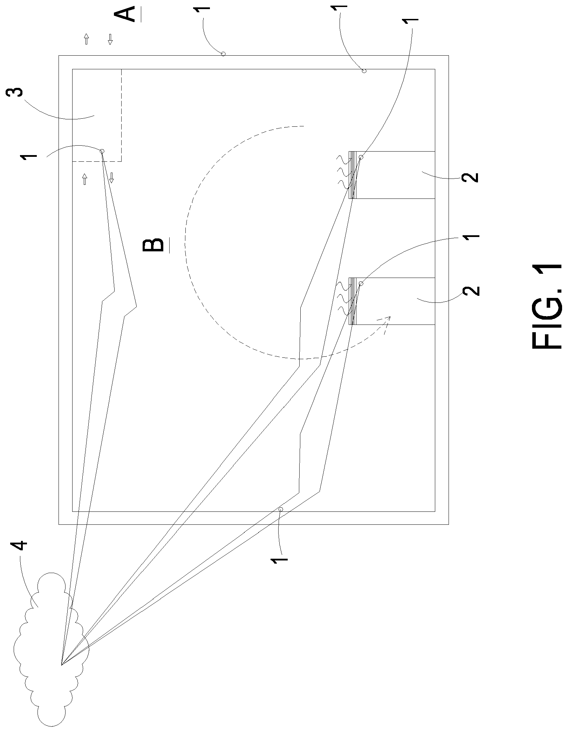

Please refer to FIG. 1. The present disclosure provides an indoor air cleaning system. The indoor air cleaning system includes a plurality of gas detectors 1, at least one cleaning device 2 and a cloud computing server 4, wherein the plurality of gas detectors 1 are disposed in an outdoor field A and an indoor field B for detecting air pollution. In the embodiment shown in FIG. 1, one gas detector 1 is disposed in the outdoor field A for detecting outdoor air pollution and outputting outdoor air pollution information via Internet of Things (IOT) communication, and multiple gas detectors 1 are disposed in the indoor field B for detecting indoor air pollution and outputting indoor air pollution information via IOT communication. The at least one cleaning device 2 includes a fan 21, a filter element 22 and a sterilization component 23. In the embodiment, the control and enablement of the fan 21 is performed via IOT communication, so as to guide the air pollution to pass through the filter element 22 for filtration, and guide the air pollution to pass through the sterilization component 23 for sterilization. Preferably but not exclusively, the fan 21 has a specific clean air delivery rate (CADR) for generating a directional circular airflow, and the filter element 22 includes a high efficiency particulate air (HEPA) filter screen. The cloud computing server 4 receives the indoor air pollution information and the outdoor air information via IOT communication, stores the air pollution information to an air pollution big data database, and performs an intelligent computing based on the big data database for intelligently selecting and issuing a control command via IOT communication to the fan 21 of the cleaning device 2 for enablement. Whereby, the fan 21 of the cleaning device 2 generates the directional circular airflow in the indoor field B (as indicated by the dotted line showing the circulating airflow path in FIG. 1), and the air pollution in the indoor field B is rapidly guided to pass through the filter element 22 multiple times for filtration to a level close to zero and pass through the sterilization component 23 for sterilization. Consequently, the gas state in the indoor field B reaches the cleanroom class by a cleanliness specification based on a passage number of particles.

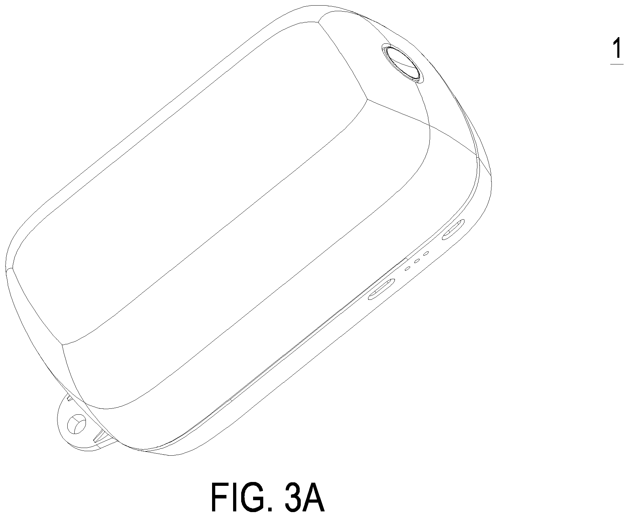

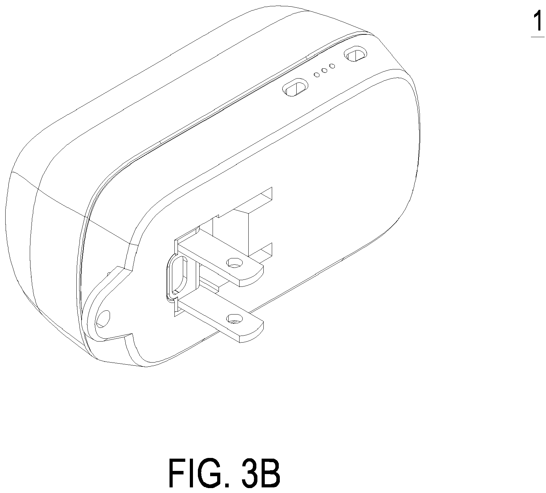

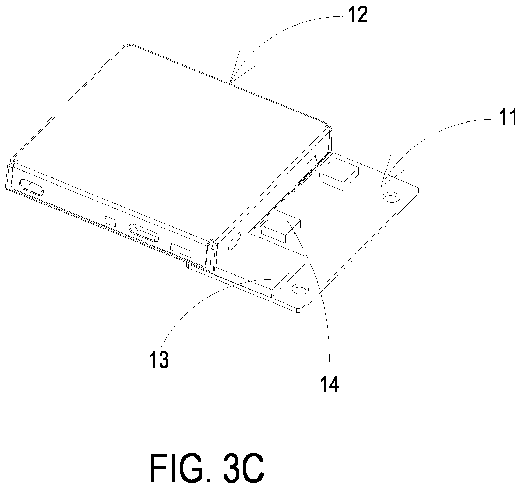

In the embodiment, the gas detector 1 includes a gas detection module installed therein. Notably, please refer to FIG. 3A and FIG. 3B. The gas detector 1 can be configured with an external power terminal, and the external power terminal can be directly inserted into the power interface in the indoor field B for enabling the detection of air pollution. Alternatively, as shown in FIG. 3C, the gas detection module without external power supply terminals is directly disposed on the device (the cleaning device 2), and connected to the power supply for enabling the detection of air pollution.

Notably, in the embodiment, the air pollution includes at least one selected from the group consisting of particulate matter, carbon monoxide, carbon dioxide, ozone, sulfur dioxide, nitrogen dioxide, lead, total volatile organic compounds (TVOC), formaldehyde, bacteria, fungi, virus and a combination thereof. The intelligent computing refers to artificial intelligent (AI) computing and edge computing. The Internet of Things (IOT) refers to the collective network that connects various devices and the technology that helps devices communicate with the cloud.

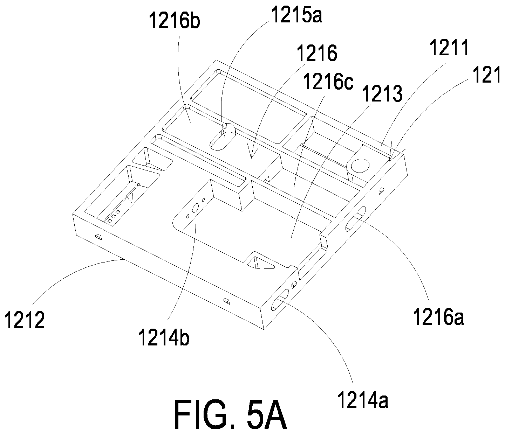

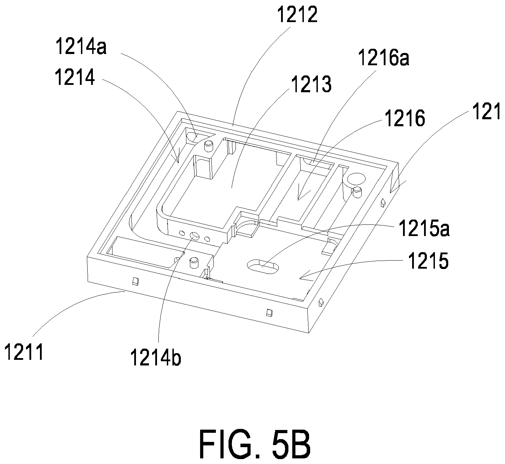

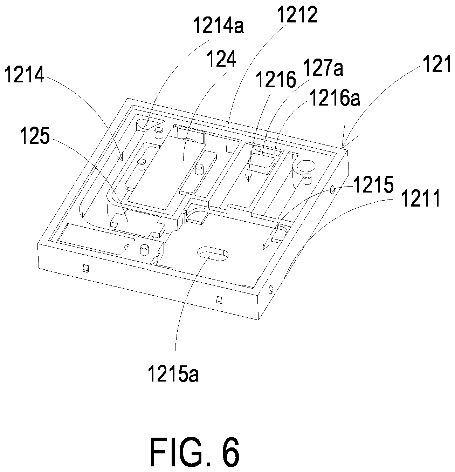

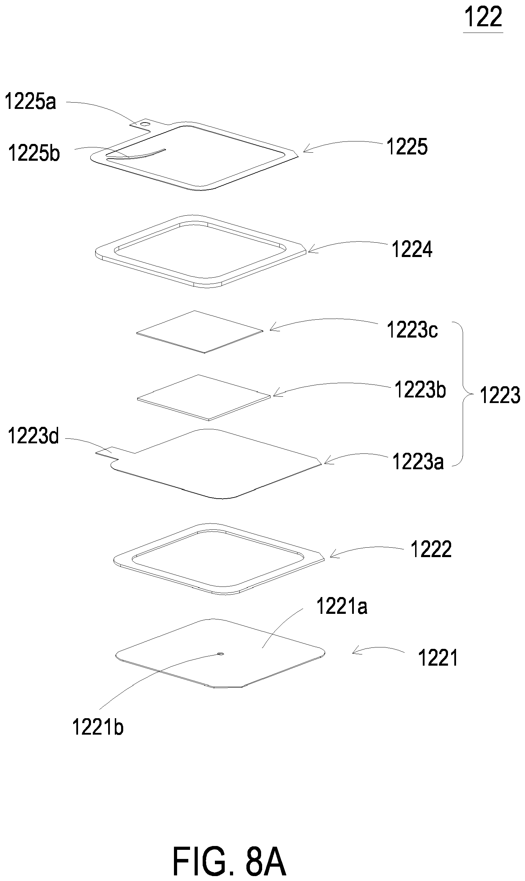

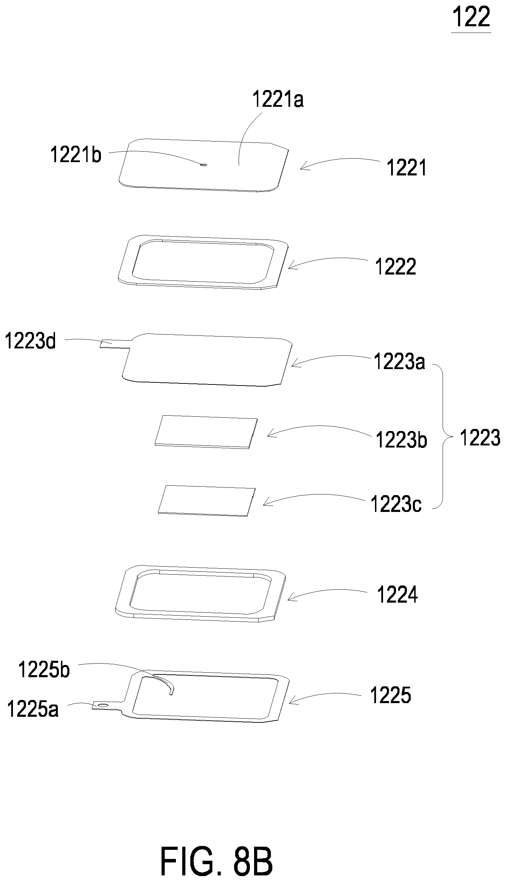

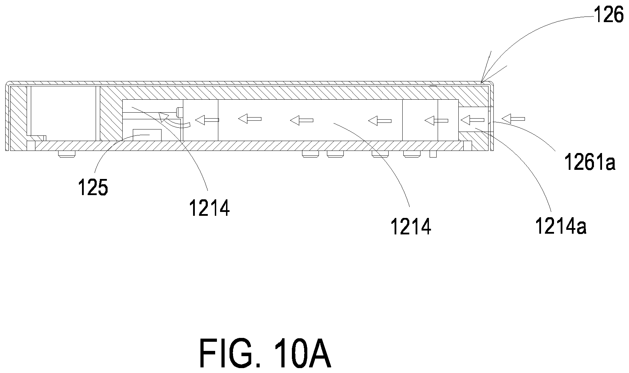

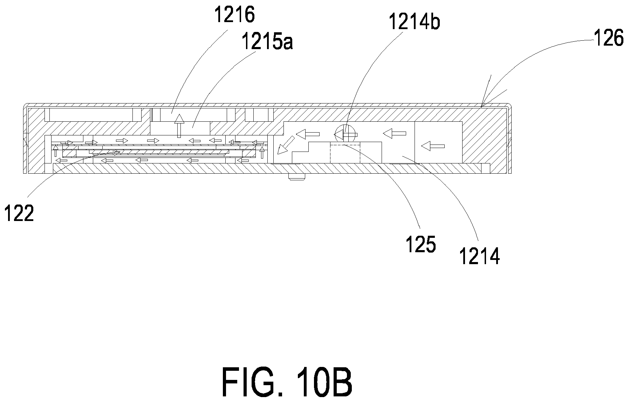

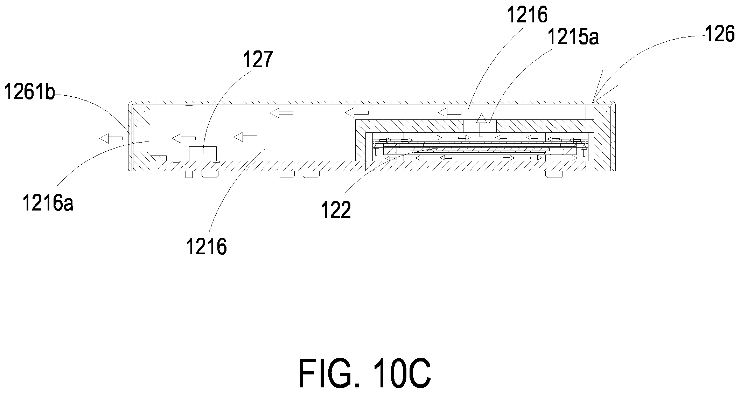

The structure of the gas detection module of the gas detector 1 of the present disclosure is described in detail below. Please refer to FIG. 3A to FIG. 11. In the embodiment, the gas detection module includes a controlling circuit board 11, a gas detection main part 12, a microprocessor 13 and a communicator 14. The gas detection main part 12, the microprocessor 13 and the communicator 14 are integrally packaged on the controlling circuit board 11 and electrically connected to each other. In the embodiment, the microprocessor 13 and the communicator 14 are mounted on the controlling circuit board 11. The microprocessor 13 controls the driving signal of the gas detection main part 12 for enabling the detection. In this way, the gas detection main part 12 detects the air pollution and outputs the air pollution information, and the microprocessor 13 receives, processes and provides the air pollution information to the communicator 14 for externally transmitting to the cloud computing server 4 via IOT communication. In the embodiment, the IOT communication refers to the mutual communication of the gas detector 1 with the cloud computing server 4 via a wireless communication. Preferably but not exclusively, the wireless communication is one selected from the group consisting of a Wi-Fi communication, a Bluetooth communication, a radio frequency identification communication and a near field communication (NFC).

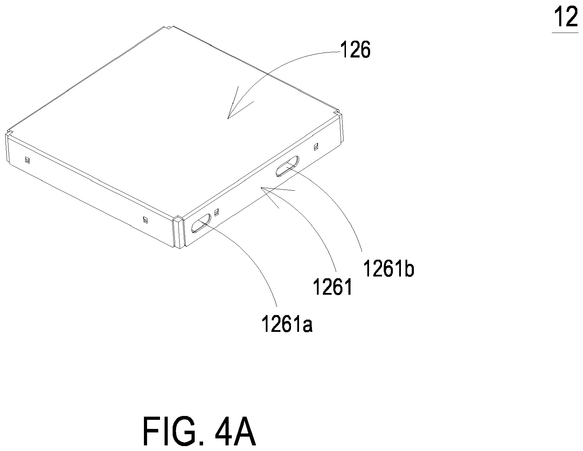

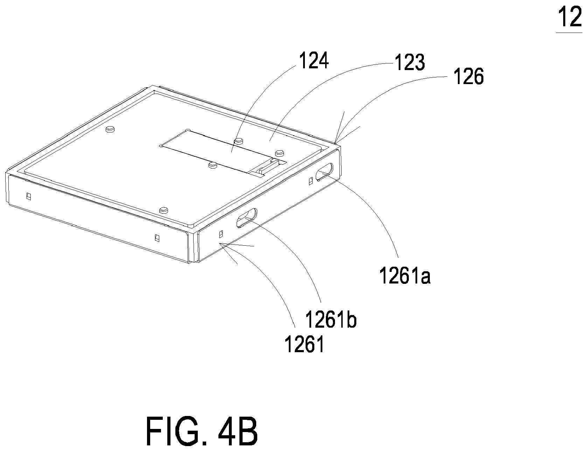

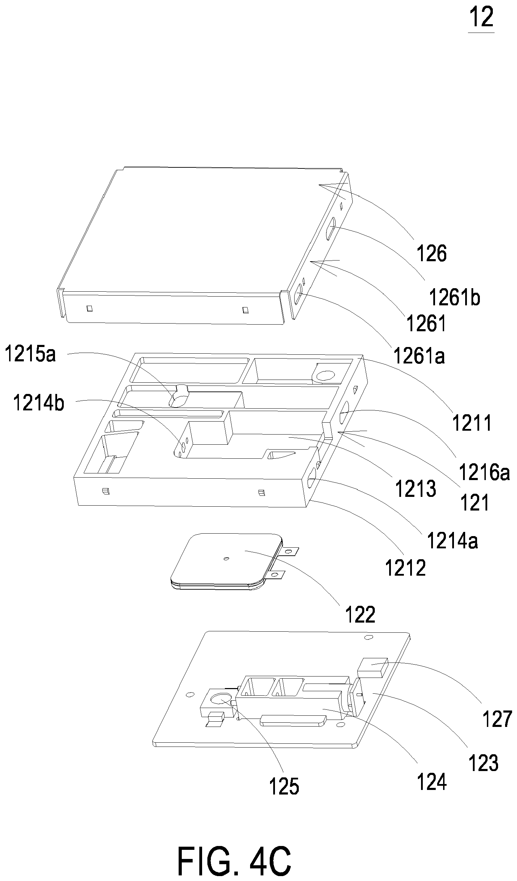

Please refer to FIG. 4A to FIG. 9A. The gas detection main part 12 includes a base 121, a piezoelectric actuator 122, a driving circuit board 123, a laser component 124, a particulate sensor 125, and an outer cover 126. In the embodiment, the base 121 includes a first surface 1211, a second surface 1212, a laser loading region 1213, a gas-inlet groove 1214, a gas-guiding-component loading region 1215 and a gas-outlet groove 1216. The first surface 1211 and the second surface 1212 are two surfaces opposite to each other. The laser loading region 1213 is hollowed out from the first surface 1211 toward the second surface 1212. The outer cover 126 covers the base 121 and includes a side plate 1261. The side plate 1261 has an inlet opening 1261a and an outlet opening 1261b. The gas-inlet groove 1214 is concavely formed from the second surface 1212 and disposed adjacent to the laser loading region 1213. The gas-inlet groove 1214 includes a gas-inlet 1214a and two lateral walls. The gas-inlet 1214a is in communication with an environment outside the base 121, and is spatially corresponding in position to an inlet opening 1261a of the outer cover 126. Two transparent windows 1214b are opened on the two lateral walls of the gas-inlet groove 1214 and are in communication with the laser loading region 1213. Therefore, the first surface 1211 of the base 121 is covered and attached by the outer cover 126, and the second surface 1212 is covered and attached by the driving circuit board 123, so that an inlet path is defined by the gas-inlet groove 1214.

In the embodiment, the gas-guiding-component loading region 1215 is concavely formed from the second surface 1212 and in communication with the gas-inlet groove 1214. A ventilation hole 1215a penetrates a bottom surface of the gas-guiding-component loading region 1215. The gas-guiding-component loading region 1215 includes four positioning protrusions 1215b disposed at four corners of the gas-guiding-component loading region 1215, respectively. In the embodiment, the gas-outlet groove 1216 includes a gas-outlet 1216a, and the gas-outlet 1216a is spatially corresponding to the outlet opening 1261b of the outer cover 126. The gas-outlet groove 1216 includes a first section 1216b and a second section 1216c. The first section 1216b is concavely formed out from the first surface 1211 in a region spatially corresponding to a vertical projection area of the gas-guiding-component loading region 1215. The second section 1216c is hollowed out from the first surface 1211 to the second surface 1212 in a region where the first surface 1211 is extended from the vertical projection area of the gas-guiding-component loading region 1215. The first section 1216b and the second section 1216c are connected to form a stepped structure. Moreover, the first section 1216b of the gas-outlet groove 1216 is in communication with the ventilation hole 1215a of the gas-guiding-component loading region 1215, and the second section 1216c of the gas-outlet groove 1216 is in communication with the gas-outlet 1216a. In that, when first surface 1211 of the base 121 is attached and covered by the outer cover 126 and the second surface 1212 of the base 121 is attached and covered by the driving circuit board 123, the gas-outlet groove 1216 and the driving circuit board 123 collaboratively define an outlet path.

In the embodiment, the laser component 124 and the particulate sensor 125 are disposed on and electrically connected to the driving circuit board 123 and located within the base 121. In order to clearly describe and illustrate the positions of the laser component 124 and the particulate sensor 125 in the base 121, the driving circuit board 123 is intentionally omitted. The laser component 124 is accommodated in the laser loading region 1213 of the base 121, and the particulate sensor 125 is accommodated in the gas-inlet groove 1214 of the base 121 and is aligned to the laser component 124. In addition, the laser component 124 is spatially corresponding to the transparent window 1214b, so that a light beam emitted by the laser component 124 passes through the transparent window 1214b and is irradiated into the gas-inlet groove 1214. A light beam path emitted from the laser component 124 passes through the transparent window 1214b and extends in an orthogonal direction perpendicular to the gas-inlet groove 1214. In the embodiment, a projecting light beam emitted from the laser component 124 passes through the transparent window 1214b and enters the gas-inlet groove 1214 to irradiate the suspended particles contained in the gas passing through the gas-inlet groove 1214. When the suspended particles contained in the gas are irradiated and generate scattered light spots, the scattered light spots are received and calculated by the particulate sensor 125, which is in an orthogonal direction perpendicular to the gas-inlet groove 1214, to obtain the gas detection information.

In the embodiment, the piezoelectric actuator 122 is accommodated in the square-shaped gas-guiding-component loading region 1215 of the base 121. In addition, the gas-guiding-component loading region 1215 is in fluid communication with the gas-inlet groove 1214. When the piezoelectric actuator 122 is enabled, the gas in the gas-inlet groove 1214 is inhaled by the piezoelectric actuator 122, so that the gas flows into the piezoelectric actuator 122, and is transported into the gas-outlet groove 1216 through the ventilation hole 1215a of the gas-guiding-component loading region 1215. Moreover, the driving circuit board 123 covers the second surface 1212 of the base 121, and the laser component 124 is positioned and disposed on the driving circuit board 123, and is electrically connected to the driving circuit board 123. The particulate sensor 125 is also positioned and disposed on the driving circuit board 123 and electrically connected to the driving circuit board 123. In that, when the outer cover 126 covers the base 121, the inlet opening 1261a is spatially corresponding to the gas-inlet 1214a of the base 121, and the outlet opening 126lb is spatially corresponding to the gas-outlet 1216a of the base 121.

In the embodiment, the piezoelectric actuator 122 includes a gas-injection plate 1221, a chamber frame 1222, an actuator element 1223, an insulation frame 1224 and a conductive frame 1225. In the embodiment, the gas-injection plate 1221 is made by a flexible material and includes a suspension plate 1221a and a hollow aperture 1221b. The suspension plate 1221a is a sheet structure and is permitted to undergo a bending deformation. Preferably but not exclusively, the shape and the size of the suspension plate 1221a are corresponding to the inner edge of the gas-guiding-component loading region 1215, but not limited thereto. The hollow aperture 1221b passes through a center of the suspension plate 1221a, so as to allow the gas to flow therethrough. Preferably but not exclusively, in the embodiment, the shape of the suspension plate 1221a is selected from the group consisting of a square, a circle, an ellipse, a triangle and a polygon, but not limited thereto.

In the embodiment, the chamber frame 1222 is carried and stacked on the gas-injection plate 1221. In addition, the shape of the chamber frame 1222 is corresponding to the gas-injection plate 1221. The actuator element 1223 is carried and stacked on the chamber frame 1222 and collaboratively defines a resonance chamber 1226 with the chamber frame 1222 and the gas-injection plate 1221. The insulation frame 1224 is carried and stacked on the actuator element 1223 and the appearance of the insulation frame 1224 is similar to that of the chamber frame 1222. The conductive frame 1225 is carried and stacked on the insulation frame 1224, and the appearance of the conductive frame 1225 is similar to that of the insulation frame 1224. In addition, the conductive frame 1225 includes a conducting pin 1225a and a conducting electrode 1225b. The conducting pin 1225a is extended outwardly from an outer edge of the conductive frame 1225, and the conducting electrode 1225b is extended inwardly from an inner edge of the conductive frame 1225. Moreover, the actuator element 1223 further includes a piezoelectric carrying plate 1223a, an adjusting resonance plate 1223b and a piezoelectric plate 1223c. The piezoelectric carrying plate 1223a is carried and stacked on the chamber frame 1222. The adjusting resonance plate 1223b is carried and stacked on the piezoelectric carrying plate 1223a. The piezoelectric plate 1223c is carried and stacked on the adjusting resonance plate 1223b. The adjusting resonance plate 1223b and the piezoelectric plate 1223c are accommodated in the insulation frame 1224. The conducting electrode 1225b of the conductive frame 1225 is electrically connected to the piezoelectric plate 1223c. In the embodiment, the piezoelectric carrying plate 1223a and the adjusting resonance plate 1223b are made by a conductive material. The piezoelectric carrying plate 1223a includes a piezoelectric pin 1223d. The piezoelectric pin 1223d and the conducting pin 1225a are electrically connected to a driving circuit (not shown) of the driving circuit board 123, so as to receive a driving signal, such as a driving frequency and a driving voltage. Through this structure, a circuit is formed by the piezoelectric pin 1223d, the piezoelectric carrying plate 1223a, the adjusting resonance plate 1223b, the piezoelectric plate 1223c, the conducting electrode 1225b, the conductive frame 1225 and the conducting pin 1225a for transmitting the driving signal. Moreover, the insulation frame 1224 is insulated between the conductive frame 1225 and the actuator element 1223, so as to avoid the occurrence of a short circuit. Thereby, the driving signal is transmitted to the piezoelectric plate 1223c. After receiving the driving signal, the piezoelectric plate 1223c deforms due to the piezoelectric effect, and the piezoelectric carrying plate 1223a and the adjusting resonance plate 1223b are further driven to generate the bending deformation in the reciprocating manner.

Furthermore, in the embodiment, the adjusting resonance plate 1223b is located between the piezoelectric plate 1223c and the piezoelectric carrying plate 1223a and served as a cushion between the piezoelectric plate 1223c and the piezoelectric carrying plate 1223a. Thereby, the vibration frequency of the piezoelectric carrying plate 1223a is adjustable. Basically, the thickness of the adjusting resonance plate 1223b is greater than the thickness of the piezoelectric carrying plate 1223a, and the vibration frequency of the actuator element 1223 can be adjusted by adjusting the thickness of the adjusting resonance plate 1223b.

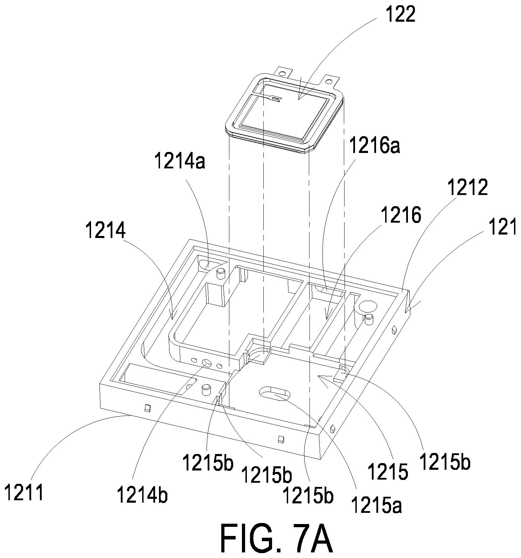

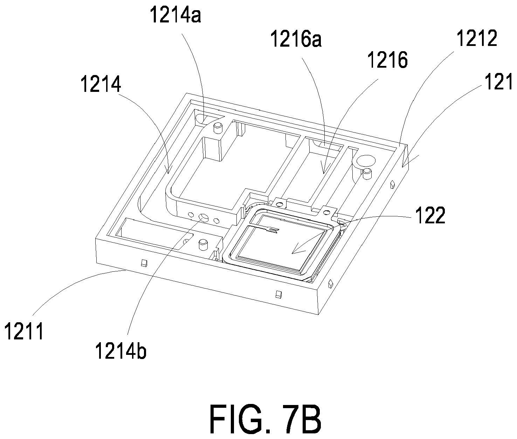

Please further refer to FIG. 7A, FIG. 7B, FIG.8A, FIG. 8B and FIG. 9A. In the embodiment, the gas-injection plate 1221, the chamber frame 1222, the actuator element 1223, the insulation frame 1224 and the conductive frame 1225 are stacked and positioned in the gas-guiding-component loading region 1215 sequentially, so that the piezoelectric actuator 122 is supported and positioned in the gas-guiding-component loading region 1215. A clearance 1221c is defined between the suspension plate 1221a and an inner edge of the gas-guiding-component loading region 1215 for gas flowing therethrough. In the embodiment, a flowing chamber 1227 is formed between the gas-injection plate 1221 and the bottom surface of the gas-guiding-component loading region 1215. The flowing chamber 1227 is in communication with the resonance chamber 1226 between the actuator element 1223, the chamber frame 1222 and the gas-injection plate 1221 through the hollow aperture 1221b of the gas-injection plate 1221. By controlling the vibration frequency of the gas in the resonance chamber 1226 to be close to the vibration frequency of the suspension plate 1221a, the Helmholtz resonance effect is generated between the resonance chamber 1226 and the suspension plate 1221a, so as to improve the efficiency of gas transportation. When the piezoelectric plate 1223c is moved away from the bottom surface of the gas-guiding-component loading region 1215, the suspension plate 1221a of the gas-injection plate 1221 is driven to move away from the bottom surface of the gas-guiding-component loading region 1215 by the piezoelectric plate 1223c. In that, the volume of the flowing chamber 1227 is expanded rapidly, the internal pressure of the flowing chamber 1227 is decreased to form a negative pressure, and the gas outside the piezoelectric actuator 122 is inhaled through the clearance 1221c and enters the resonance chamber 1226 through the hollow aperture 1221b. Consequently, the pressure in the resonance chamber 1226 is increased to generate a pressure gradient. When the suspension plate 1221a of the gas-injection plate 1221 is driven by the piezoelectric plate 1223c to move toward the bottom surface of the gas-guiding-component loading region 1215, the gas in the resonance chamber 1226 is discharged out rapidly through the hollow aperture 1221b, and the gas in the flowing chamber 1227 is compressed, thereby the converged gas is quickly and massively ejected out of the flowing chamber 1227 under the condition close to an ideal gas state of the Benulli's law, and transported to the ventilation hole 1215a of the gas-guiding-component loading region 1215.

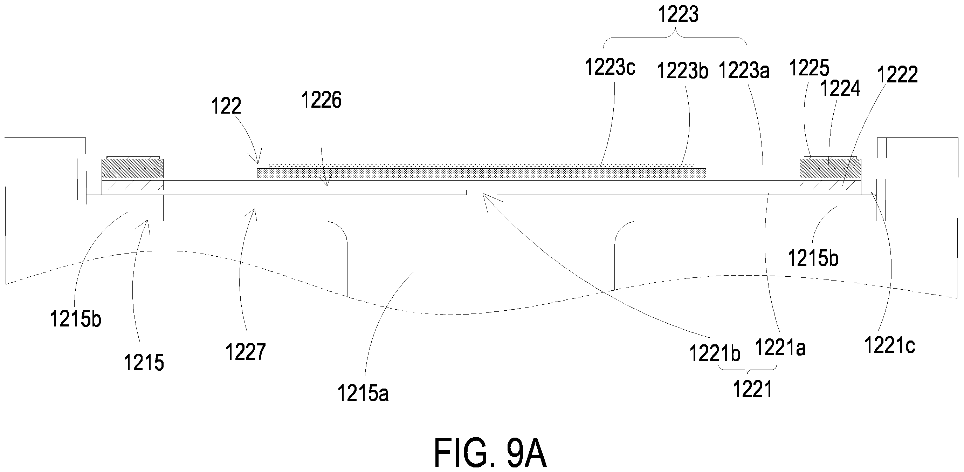

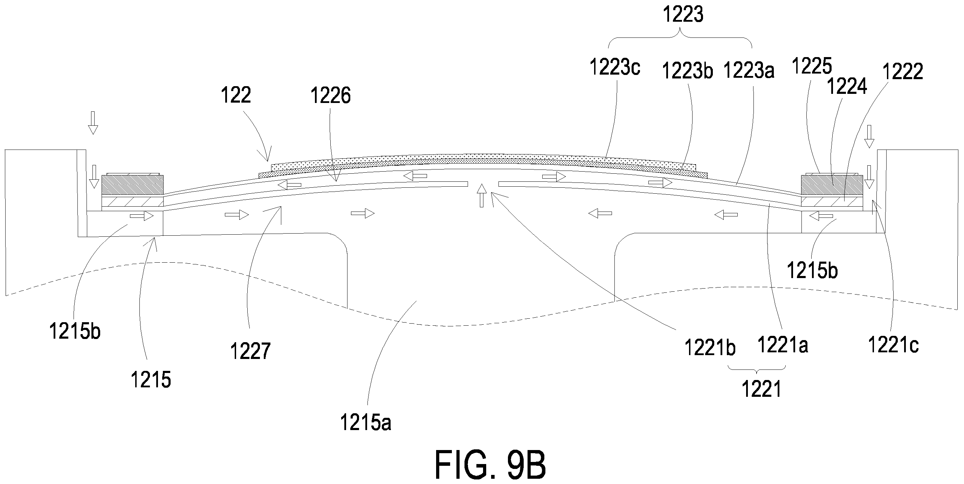

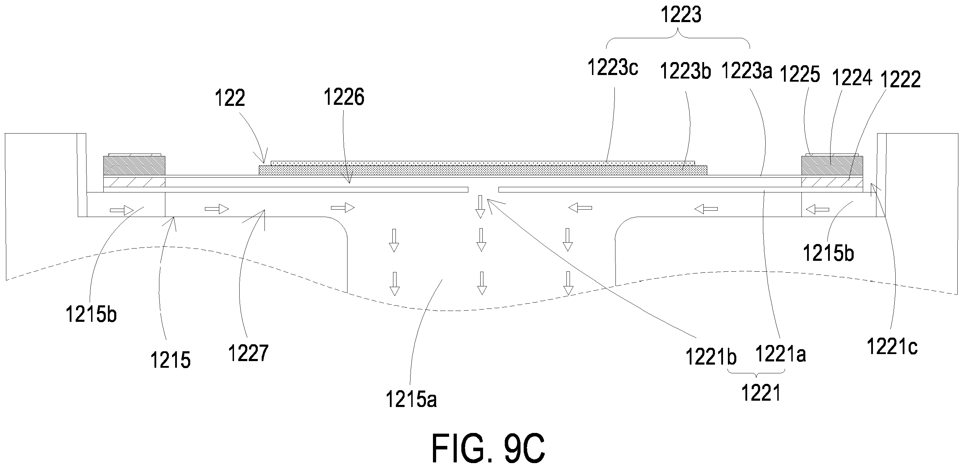

By repeating the above operation steps shown in FIG. 9B and FIG. 9C, the piezoelectric plate 1223c is driven to generate the bending deformation in a reciprocating manner. According to the principle of inertia, since the gas pressure inside the resonance chamber 1226 is lower than the equilibrium gas pressure after the converged gas is ejected out, the gas is introduced into the resonance chamber 1226 again. Moreover, the vibration frequency of the gas in the resonance chamber 1226 is controlled to be close to the vibration frequency of the piezoelectric plate 1223c, so as to generate the Helmholtz resonance effect to achieve the gas transportation at high speed and in large quantities. The gas is inhaled through the gas-inlet 1214a on the outer cover 126, flows into the gas-inlet groove 1214 of the base 121 through the gas-inlet 1214a, and is transported to the position of the particulate sensor 125. The piezoelectric actuator 122 is enabled continuously to inhale the gas into the inlet path, and facilitate the gas outside the gas detection module to be introduced rapidly, flow stably, and transported above the particulate sensor 125. At this time, a projecting light beam emitted from the laser component 124 passes through the transparent window 1214b to irritate the suspended particles contained in the gas flowing above the particulate sensor 125 in the gas-inlet groove 1214. When the suspended particles contained in the gas are irradiated and generate scattered light spots, the scattered light spots are received and calculated by the particulate sensor 125 for obtaining related information about the sizes and the concentration of the suspended particles contained in the gas. Moreover, the gas above the particulate sensor 125 is continuously driven and transported by the piezoelectric actuator 122, flows into the ventilation hole 1215a of the gas-guiding-component loading region 1215, and is transported to the gas-outlet groove 1216. At last, after the gas flows into the gas outlet groove 1216, the gas is continuously transported into the gas-outlet groove 1216 by the piezoelectric actuator 122, and thus the gas in the gas-outlet groove 1216 is pushed to discharge through the gas-outlet 1216a and the outlet opening 1261b.

The gas detector 1 of the present disclosure not only can detect the particulate matters in the gas, but also can detect the gas characteristics of the introduced gas, for example, to determine whether the gas is formaldehyde, ammonia, carbon monoxide, carbon dioxide, oxygen, ozone, or the like. Therefore, in one or some embodiments, the gas detector 1 of the present disclosure further includes a gas sensor 127 positioned and disposed on the driving circuit board 123, electrically connected to the driving circuit board 123, and accommodated in the gas-outlet groove 1216, so as to detect the gas characteristics of the introduced gas. Preferably but not exclusively, in an embodiment, the gas sensor 127 includes a volatile-organic-compound sensor for detecting the information of carbon dioxide (CO₂) or volatile organic compounds (TVOC). Preferably but not exclusively, in an embodiment, the gas sensor 127 includes a formaldehyde sensor for detecting the information of formaldehyde (HCHO) gas. Preferably but not exclusively, in an embodiment, the gas sensor 127 includes a bacteria sensor for detecting the information of bacteria or fungi. Preferably but not exclusively, in an embodiment, the gas sensor 127 includes a virus sensor for detecting the information of virus in the gas. Preferably but not exclusively, the gas sensor 127 is a temperature and humidity sensor for detecting the temperature and humidity information of the gas.

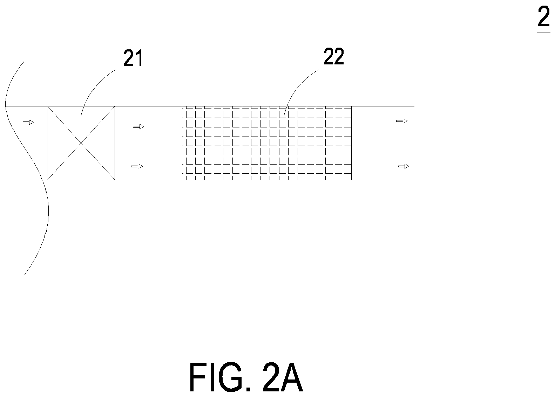

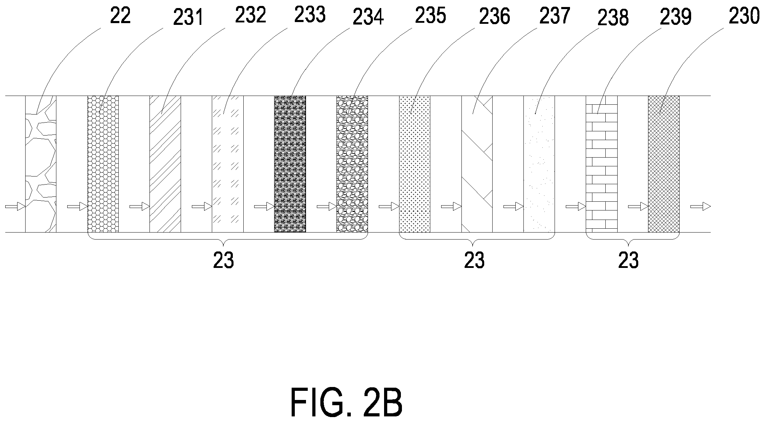

Please refer to FIG. 1, FIG. 2A and FIG. 2B. In the embodiment, the cleaning device 2 includes a fan 21, a filter element 22 and a sterilization component 23. The fan 21 is controlled and enabled to guide the air pollution to pass through the filter element 22 for filtration, and guide the air pollution to pass through the sterilization component 23 for sterilization. In the embodiment, the filter element 22 includes a high efficiency particulate air (HEPA) filter screen, which is configured to absorb the chemical smoke, the bacteria, the dust particles and the pollen contained in the air pollution, so that the air pollution introduced into the filter element 22 is filtered and purified to achieve the effect of filtering and purification. As shown in FIG. 2A, the airflow of the fan 21 flows in the path indicated by the arrows. The fan 21 can be arranged at the front side of the filter element 22, and the fan 21 can also be arranged at the rear side of the filter element 22. Notably, in the embodiment, the sterilization component 23 is combined with the filter element 22 and sterilizes in chemical means through coating a decomposition layer. Preferably but not exclusively, the decomposition layer includes an activated carbon 231 configured to remove organic and inorganic substances in the air pollution, and remove colored and odorous substances. Preferably but not exclusively, the decomposition layer includes a cleansing factor containing chlorine dioxide layer 232 configured to inhibit viruses, bacteria, fungi, influenza A, influenza B, enterovirus and norovirus in the air pollution, and the inhibition ratio can reach 99% and more, thereby reducing the cross-infection of viruses. Preferably but not exclusively, the decomposition layer includes an herbal protective layer 233 extracted from ginkgo and Japanese Rhus chinensis configured to resist allergy effectively and destroy a surface protein of influenza virus (such as H1N1 influenza virus) passing therethrough. Preferably but not exclusively, the decomposition layer includes a silver ion 234 configured to inhibit viruses, bacteria and fungi contained in the air pollution. Preferably but not exclusively, the decomposition layer includes a zeolite 235 configured to remove ammonia nitrogen, heavy metals, organic pollutants, Escherichia coli, phenol, chloroform and anionic surfactants. Furthermore, in some embodiments, the sterilization component 23 includes a light irradiation element combined with the filter element 22 to sterilize in chemical means. Preferably but not exclusively, the light irradiation element is a photo-catalyst unit including a photo catalyst 236 and an ultraviolet lamp 237. When the photo catalyst 236 is irradiated by the ultraviolet lamp 237, the light energy is converted into the electrical energy, thereby decomposing harmful substances and disinfects bacteria contained in the air pollution, so as to achieve the effects of filtering and purification. Preferably but not exclusively, the light irradiation element is a photo-plasma unit including a nanometer irradiation tube 238. When the introduced air pollution is irradiated by the nanometer irradiation tube 238, the oxygen molecules and water molecules contained in the air pollution are decomposed into high oxidizing photo-plasma, and an ion flow capable of destroying organic molecules is generated. In that, volatile formaldehyde, volatile toluene and volatile organic compounds (VOC) contained in the air pollution are decomposed into water and carbon dioxide, so as to achieve the effects of filtering and purification. Moreover, in some embodiments, the sterilization component 23 includes a decomposition unit combined with the filter element 22 to sterilize in chemical means. Preferably but not exclusively, the decomposition unit is a negative ion unit 239 with a dust collecting plate. It makes the suspended particles in the air pollution to carry with positive charge and adhere to the dust collecting plate carry with negative charges, so as to achieve the effects of filtering and purification. Preferably but not exclusively, the decomposition unit is a plasma ion unit 230. The oxygen molecules and water molecules contained in the air pollution are decomposed into positive hydrogen ions (H⁺) and negative oxygen ions (O²⁻) by the plasma ion. The substances attached with water around the ions are adhered on the surfaces of viruses and bacteria and converted into OH radicals with extremely strong oxidizing power, thereby removing hydrogen (H) from the protein on the surfaces of viruses and bacteria, and thus decomposing (oxidizing) the protein, so as to filter the introduced air pollution and achieve the effects of filtering and purification.

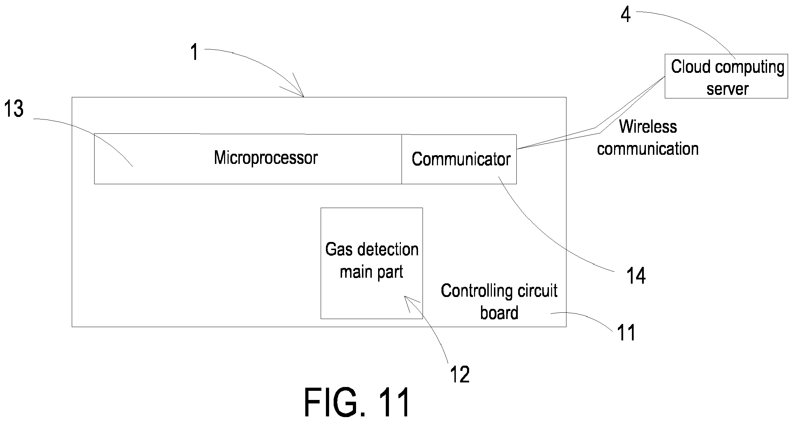

Please refer to FIG. 1 and FIG. 11. The gas detector 1 can be directly installed in the cleaning device 2, and the gas detection module of the gas detector 1 is configured to connect the power supply and the controlling and driving circuit of the cleaning device 2, so that the communicator 14 of the gas detection module and the cloud computing server 4 can communicate with each other via IOT communication, and the cloud computing server 4 can control the enablement of the fan 21 of the cleaning device 2 through issuing the control command. In the embodiment, the enablement is based on the air pollution information detected in the indoor field B. Through the cloud computing server 4 performs the intelligent computing for comparison based on the air pollution big data database, the enabling cycle and the air volume of the fan 21 of the cleaning device 2 is adjusted to generate the directional circular airflow in the indoor field B to guide the air pollution to rapidly pass through the filter element 22 multiple times for filtration and pass through the sterilization component 23 for sterilization, so that the purification efficiency to a level close to zero is improved and the environmental noises in the indoor field B is reduced. In some embodiments, the indoor field B is disposed with a plurality of gas detectors 1 and a plurality of cleaning devices 2. In the embodiment, at least two of the gas detectors 1 detect and output the air pollution information to the cloud computing server 4 via IOT communication. The cloud computing server 4 performs the intelligent computing to locate an air pollution position in the indoor field B and intelligently issues the control command to the plurality of cleaning devices 2 for enabling the fan 21 of one of the plurality of cleaning devices 2 which is near the air pollution position to generate a directional airflow and then enabling the fans 21 of others cleaning devices 2, thereby generating the directional circular airflow, so that the air pollution is rapidly guided to pass through the filter element 22 multiple times for filtration to a level close to zero and through the sterilization component 23 for sterilization, and the gas state in the indoor field B reaches the cleanroom class by a cleanliness specification based on the passage number of particles.

Please refer to FIG. 1. The present disclosure further includes a gas exchanging device 3 for guiding the air from the outdoor field A to the indoor field B so as to achieve a gas exchanging. The flowing paths of air between the outdoor field A and the indoor field B are indicated by arrows shown in FIG. 1. The gas detector 1 can be directly disposed on the gas exchanging device 3, and the gas detection module of the gas detector 1 is configured to connect the power supply and the controlling and driving circuit of the gas exchanging device 3, so that the communicator 14 of the gas detection module and the cloud computing server 4 can communicate with each other via IOT communication. In the embodiment, the enablement of the gas exchanging device 3 is controlled by the control command issued from the cloud computing server 4 via IOT communication, that is, through intelligently comparing the air pollution information between the outdoor field A and the indoor field B, when the air pollution information of the indoor field B is greater than the air pollution information of the outdoor field A, the cloud computing server 4 issues the control command via IOT communication to enable the gas exchanging device 3 to guide the air from the outdoor field A to the indoor field B for gas exchanging. In this way, the indoor field B is maintained in a positive pressure gas-exchanging state, and the air pollution will not remain in the indoor field B. Furthermore, through the continuous gas exchanging and the directional circular airflow generated by the plurality of cleaning devices 2, the air pollution in the indoor field B can be rapidly guided to pass through the filter element 22 multiple times for filtration. When the indoor air pollution information is lower than the outdoor air pollution information, the cloud computing server 4 again issues the control command to the gas exchanging device 3 via IOT communication to disable the gas exchanging device 3. Accordingly, the gas state in the indoor field B is again controlled by the directional circular airflow generated by the plurality of cleaning devices 2, where the air pollution in the indoor field B is rapidly guided to pass through the filter element 22 multiple times for filtration to a level close to zero and to pass through the sterilization component 23 for sterilization, thereby reaching the gas state in the indoor field B to the classroom class by the cleanliness specification based on the passage number of particles. Notably, the gas exchanging device 3 includes a fresh air fan.

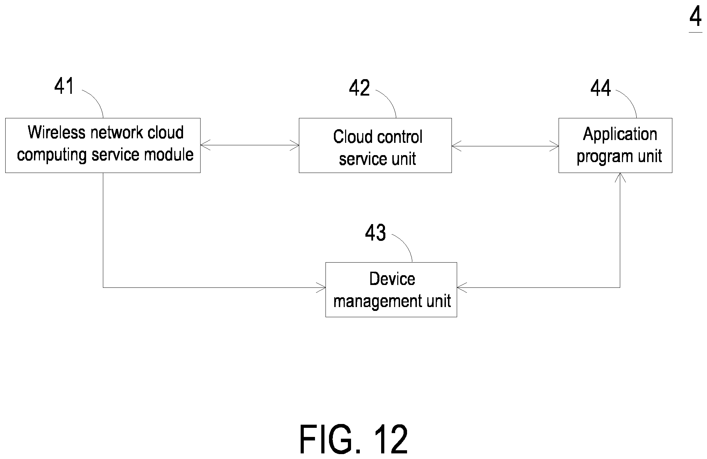

Please refer to FIG. 12. In the embodiment, the cloud computing server 4 includes a wireless network cloud computing service module 41, a cloud control service unit 42, a device management unit 43 and an application program unit 44. The wireless network cloud computing service module 41 receives the outdoor air pollution information of the outdoor field A and the indoor air pollution information of the indoor field B, receives communication information of the cleaning device 2 and the gas exchanging device 3, and transmits a first control command and a second control command. Moreover, the wireless network cloud computing service module 41 receives the outdoor air pollution information of the outdoor field A and the indoor air pollution information of the indoor field B and transmits thereof to the cloud control service unit 42 to store to and form an air pollution big data database. The intelligence computing and comparison based on the air pollution big data database are implemented to determine the location of the air pollution, and accordingly, the control command is transmitted to the wireless network cloud computing service module 41, and then transmitted to the gas exchanging device 3 and the fan 21 of the cleaning device 2 to control the enablement thereof through the wireless network cloud computing service module 41. The device management unit 43 receives the communication information of the cleaning device 2 and the gas exchanging device 3 through the wireless network cloud computing service module 41 to manage the user login and device binding, and the device management information can be provided to the application program unit 44 for system control and management. The application program unit 44 can also display and inform the air pollution information obtained from the cloud control service unit 42, so the user can know the real-time status of air pollution removal through the mobile phone or the communication device. Moreover, the user can control the operation of the indoor air cleaning system through the application program unit 44 of the mobile phone or the communication device.

In view of the above description of the present disclosure, a plurality of gas detectors 1 and at least one cleaning device 2 are disposed in the outdoor field A and the indoor field B, so that the gas detectors 1 can monitor and determine the air pollution at any time, and output the air pollution information of the outdoor field A and the indoor field B. Then, the air pollution information is received through the cloud computing server 4 and stored in the air pollution big data database, and the intelligence computing is implemented to determine the location of air pollution. Furthermore, a control command is intelligently issued to the gas exchanging device 3 via IOT communication for enabling the gas exchanging device 3 to guide the air from the outdoor field A to the indoor field B for gas exchanging, and a control command is issued to the fan 21 of the cleaning device 2 via IOT communication for enabling the fan 21 to generate the directional circular airflow in the indoor field B for rapidly guiding the air pollution to pass through the filter element 22 multiple times for filtration to a level close to zero and pass through the sterilization component 23 for sterilization, thereby reaching the gas state in the indoor field B to the cleanroom class by the cleanliness specification based on the passage number of particles.

Notably, an air pollution safety detection value is determined through the stored air pollution information and the intelligent computing of the cloud computing server 4, and then, based thereon, the control command is issued to the gas exchanging device 3 for enablement, and the control command is also issued to the cleaning device 2 for enablement. Preferably but not exclusively, the air pollution safety detection value includes at least one selected from the group consisting of a concentration of PM2.5 which is less than or equal to 10 µg/m³, a concentration of carbon dioxide (CO₂) which is less than or equal to 1000 ppm, a concentration of total volatile organic compounds (TVOC) which is less than or equal to 0.56 ppm, a concentration of formaldehyde (HCHO) which is less than or equal to 0.08 ppm, a colony-forming unit of bacteria which is less than or equal to 1500 CFU/m³, a colony-forming unit of fungi which is less than or equal to 1000 CFU/m³, a concentration of sulfur dioxide which is less than or equal to 0.075 ppm, a concentration of nitrogen dioxide which is less than or equal to 0.1 ppm, a concentration of carbon monoxide which is less than or equal to 9 ppm, a concentration of ozone which is less than or equal to 0.06 ppm, and a concentration of lead which is less than or equal to 0.15 µg/m³.

Certainly, the indoor air cleaning system of the present disclosure can be applied to the indoor field B of home and office. In order to make the gas state of the indoor field B reach the cleanliness of the cleanroom class, different from the clean rooms used in the general semiconductor industry, the hospitals or the biotechnology plants, the filter element 22 of the cleaning device 2 filters the air pollution with the HEPA filter screen, and the fan 21 of the cleaning device 2 has a specific clean air delivery rate (CADR). Whereby, the directional circular airflow is generated continuously in the indoor field B, and the air pollution is rapidly guided to pass through the filter element 22 multiple times for filtration to a level close to zero and through the sterilization component 23 for sterilization, so that the gas state in the indoor space field B can meet the requirements of the cleanroom class by the cleanliness specification based on the passage number of particles, and the impact and injury of human health caused by the gas hazards in the environment can be avoided. It allows the indoor field B of home and office to achieve the required cleanliness of cleanroom class at a price lower than 1/4~1/10 of the cost of setting up the clean room in the general semiconductor industry, the hospitals or the biotechnology plants, which is extremely industrially applicable.

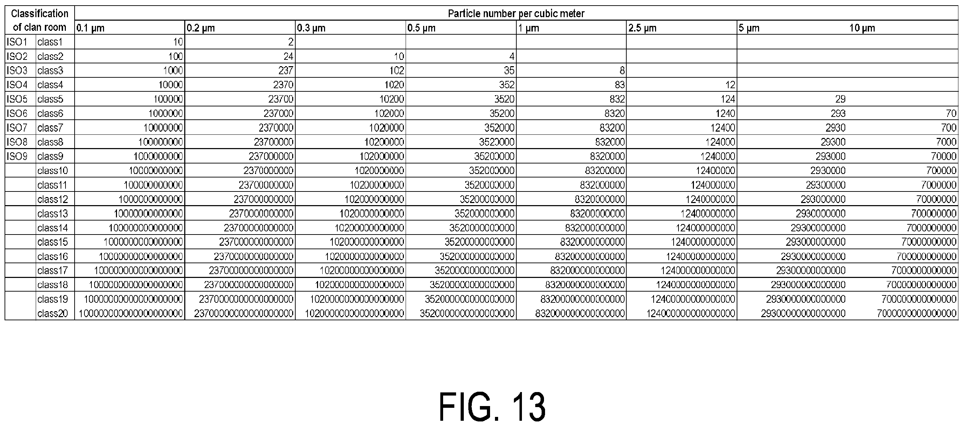

In the following, several specific embodiments are described. As shown in FIG. 13, the present disclosure formulates a comparison table of cleanliness specifications of cleanroom classes which are based on the passage number of particles for the air pollution in the indoor field B, so as to illustrate the number of suspended particles per cubic meter in the air pollution of the indoor field B used to distinguish the classifications of cleanroom classes achieved by the air cleaning system of the present disclosure. Notably, the cleanroom class 1 to class 9 in the comparison table are completely the same as the ISO standard cleanroom class 1 to class 9, and the cleanroom class 10 to class 20 are also standardized.

In a specific embodiment, the fan 21 has a clean air delivery rate (CADR) greater than CADR 1500, the filter element 22 includes a nanometer filter, and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10², and a passage number of particles in size of 0.2 µm less than or equal to 2, thereby achieving the cleanliness of cleanroom class 1 (CLASS 1). Notably, the nanometer filter is one selected from the group consisting of a nano fiber, a nano activated carbon, a nano film and a combination thereof.

In a specific embodiment, the fan 21 has a clean air delivery rate (CADR) greater than CADR 1500, the filter element 22 includes an ultra low particulate air (ULPA) filter of U17 (ULPA 17), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm is less than or equal to 1×10², a passage number of particles in size of 0.2 µm is less than or equal to 24, a passage number of particles in size of 0.3 µm is less than or equal to 10, and a passage number of particles in size of 0.5 µm size is less than or equal to 4, thereby achieving the cleanliness of cleanroom class 2 (CLASS 2).

In a specific embodiment, the fan 21 has a clean air delivery rate (CADR) greater than CADR 1000, the filter element 22 includes an ultra low particulate air (ULPA) filter of U16 (ULPA 16), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10³, a passage number of particles in size of 0.2 µm less than or equal to 237, a passage number of particles in size of 0.3 µm less than or equal to 102, a passage number of particles in size of 0.5 µm size less than or equal to 35, and a passage number of particles in size of 1 µm less than or equal to 8, thereby achieving the cleanliness of cleanroom class 3 (CLASS 3).

In a specific embodiment, the fan 21 has a clean air delivery rate (CADR) greater than CADR 1000, the filter element 22 includes an ultra low particulate air (ULPA) filter of U15 (ULPA 15), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10⁴, a passage number of particles in size of 0.2 µm less than or equal to 237×10¹, a passage number of particles in size of 0.3 µm less than or equal to 1 02x 101, a passage number of particles in size of 0.5 µm size less than or equal to 352, a passage number of particles in size of 1 µm less than or equal to 83, and a passage number of particles in size of 2.5 µm size less than or equal to 12, thereby achieving the cleanliness of cleanroom class 4 (CLASS 4).

In a specific embodiment, the fan 21 has a clean air delivery rate (CADR) greater than CADR 1000, the filter element 22 includes an ultra low particulate air (ULPA) filter of U15 (ULPA 15), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10⁵, a passage number of particles in size of 0.2 µm less than or equal to 237×10², a passage number of particles in size of 0.3 µm less than or equal to 102×10², a passage number of particles in size of 0.5 µm size less than or equal to 352×10¹, a passage number of particles in size of 1 µm less than or equal to 832, a passage number of particles in size of 2.5 µm size less than or equal to 124, and a passage number of particles in size of 5 µm size less than or equal to 29, thereby achieving the cleanliness of cleanroom class 5 (CLASS 5).

In a specific embodiment, the fan 21 has a clean air delivery rate (CADR) greater than CADR 600, the filter element 22 includes a high efficiency particulate air (HEPA) filter of H14 (HEPA 14), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10⁶, a passage number of particles in size of 0.2 µm less than or equal to 237×10³, a passage number of particles in size of 0.3 µm less than or equal to 102×10³, a passage number of particles in size of 0.5 µm size less than or equal to 352×10², a passage number of particles in size of 1 µm less than or equal to 832×10¹, a passage number of particles in size of 2.5 µm size less than or equal to 124×10¹, a passage number of particles in size of 5 µm size less than or equal to 293, and a passage number of particles in size of 10 µm size less than or equal to 70, thereby achieving the cleanliness of cleanroom class 6 (CLASS 6).

In a specific embodiment, the fan 21 has a clean air delivery rate (CADR) greater than CADR 600, the filter element 22 includes a high efficiency particulate air (HEPA) filter of H13 (HEPA 13), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10⁷, a passage number of particles in size of 0.2 µm less than or equal to 237×10⁴, a passage number of particles in size of 0.3 µm less than or equal to 102×10⁴, a passage number of particles in size of 0.5 µm size less than or equal to 352×10³, a passage number of particles in size of 1 µm less than or equal to 832×10², a passage number of particles in size of 2.5 µm size less than or equal to 124×10², a passage number of particles in size of 5 µm size less than or equal to 293×10¹, and a passage number of particles in size of 10 µm size less than or equal to 70×10¹, thereby achieving the cleanliness of cleanroom class 7 (CLASS 7).

In a specific embodiment, the fan 21 has a clean air delivery rate (CADR) greater than CADR 600, the filter element 22 includes a high efficiency particulate air (HEPA) filter of H13 (HEPA 13), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10⁸, a passage number of particles in size of 0.2 µm less than or equal to 237×10⁵, a passage number of particles in size of 0.3 µm less than or equal to 102×10⁵, a passage number of particles in size of 0.5 µm size less than or equal to 352×10⁴, a passage number of particles in size of 1 µm less than or equal to 832×10³, a passage number of particles in size of 2.5 µm size less than or equal to 124×10³, a passage number of particles in size of 5 µm size less than or equal to 293×10², and a passage number of particles in size of 10 µm size less than or equal to 70×10², thereby achieving the cleanliness of cleanroom class 8 (CLASS 8).

In a specific embodiment, the fan 21 has a clean air delivery rate (CADR) greater than CADR 600, the filter element 22 includes a high efficiency particulate air (HEPA) filter of H12 (HEPA 12), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10⁹, a passage number of particles in size of 0.2 µm less than or equal to 237×10⁶, a passage number of particles in size of 0.3 µm less than or equal to 102×10⁶, a passage number of particles in size of 0.5 µm size less than or equal to 352×10⁵, a passage number of particles in size of 1 µm less than or equal to 832×10², a passage number of particles in size of 2.5 µm size less than or equal to 124×10⁴, a passage number of particles in size of 5 µm size less than or equal to 293×10³, and a passage number of particles in size of 10 µm size less than or equal to 70×10³, thereby achieving the cleanliness of cleanroom class 9 (CLASS 9)

In a specific embodiment, the fan 21 has a clean air delivery rate (CADR) less than CADR 600, the filter element 22 includes a high efficiency particulate air (HEPA) filter of H12 (HEPA 12), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10¹⁰, a passage number of particles in size of 0.2 µm less than or equal to 237×10⁷, a passage number of particles in size of 0.3 µm less than or equal to 102×10⁷, a passage number of particles in size of 0.5 µm size less than or equal to 352×10⁶, a passage number of particles in size of 1 µm less than or equal to 832×10⁵, a passage number of particles in size of 2.5 µm size less than or equal to 124×10⁵, a passage number of particles in size of 5 µm size less than or equal to 293×10⁴, and a passage number of particles in size of 10 µm size less than or equal to 70×10⁴, thereby achieving the cleanliness of cleanroom class 10 (CLASS 10).

In a specific embodiment, the fan 21 has a clean air delivery rate (CADR) less than CADR 600, the filter element 22 includes a high efficiency particulate air (HEPA) filter of H12 (HEPA 12), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10¹¹, a passage number of particles in size of 0.2 µm less than or equal to 237×10⁸, a passage number of particles in size of 0.3 µm less than or equal to 102×10⁸, a passage number of particles in size of 0.5 µm size less than or equal to 352×10⁷, a passage number of particles in size of 1 µm less than or equal to 832×10⁶, a passage number of particles in size of 2.5 µm size less than or equal to 124×10⁶, a passage number of particles in size of 5 µm size less than or equal to 293×10⁵, and a passage number of particles in size of 10 µm size less than or equal to 70×10⁵, thereby achieving the cleanliness of cleanroom class 11 (CLASS 11).

In a specific embodiment, the fan 21 has a clean air delivery rate (CADR) less than CADR 600, the filter element 22 includes a high efficiency particulate air (HEPA) filter of H12 (HEPA 12), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10¹², a passage number of particles in size of 0.2 µm less than or equal to 237×10⁹, a passage number of particles in size of 0.3 µm less than or equal to 102×10⁹, a passage number of particles in size of 0.5 µm size less than or equal to 352×10⁸, a passage number of particles in size of 1 µm less than or equal to 832×10⁷, a passage number of particles in size of 2.5 µm size less than or equal to 124×10⁷, a passage number of particles in size of 5 µm size less than or equal to 293×10⁶, and a passage number of particles in size of 10 µm size less than or equal to 70×10⁶, thereby achieving the cleanliness of cleanroom class 12 (CLASS 12).

In a specific embodiment, the fan 21 has a clean air delivery rate (CADR) less than CADR 600, the filter element 22 includes a high efficiency particulate air (HEPA) filter of H12 (HEPA 12), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10¹³, a passage number of particles in size of 0.2 µm less than or equal to 237×10¹⁰, a passage number of particles in size of 0.3 µm less than or equal to 102×10¹⁰, a passage number of particles in size of 0.5 µm size less than or equal to 352×10⁹, a passage number of particles in size of 1 µm less than or equal to 832×10⁸, a passage number of particles in size of 2.5 µm size less than or equal to 124×10⁸, a passage number of particles in size of 5 µm size less than or equal to 293×10⁷, and a passage number of particles in size of 10 µm size less than or equal to 70×10⁷, thereby achieving the cleanliness of cleanroom class 13 (CLASS 13).

In a specific embodiment, the fan 21 has a clean air delivery rate (CADR) less than CADR 600, the filter element 22 includes a high efficiency particulate air (HEPA) filter of H12 (HEPA 12), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10¹⁴, a passage number of particles in size of 0.2 µm less than or equal to 237×10¹¹, a passage number of particles in size of 0.3 µm less than or equal to 102×10¹¹, a passage number of particles in size of 0.5 µm size less than or equal to 352×10¹⁰, a passage number of particles in size of 1 µm less than or equal to 832×10⁹, a passage number of particles in size of 2.5 µm size less than or equal to 124×10⁹, a passage number of particles in size of 5 µm size less than or equal to 293×10⁸, and a passage number of particles in size of 10 µm size less than or equal to 70×10⁸, thereby achieving the cleanliness of cleanroom class 14 (CLASS 14).

In a specific embodiment, the fan 21 has a clean air delivery rate (CADR) less than CADR 600, the filter element 22 includes a high efficiency particulate air (HEPA) filter of H12 (HEPA 12), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10¹⁵, a passage number of particles in size of 0.2 µm less than or equal to 237×10¹², a passage number of particles in size of 0.3 µm less than or equal to 102×10¹², a passage number of particles in size of 0.5 µm size less than or equal to 352×10¹¹, a passage number of particles in size of 1 µm less than or equal to 832×10¹⁰, a passage number of particles in size of 2.5 µm size less than or equal to 124×10¹⁰, a passage number of particles in size of 5 µm size less than or equal to 293×10⁹, and a passage number of particles in size of 10 µm size less than or equal to 70×10⁹, thereby achieving the cleanliness of cleanroom class 15 (CLASS 15).

In a specific embodiment, the fan 21 has a clean air delivery rate (CADR) less than CADR 600, the filter element 22 includes a high efficiency particulate air (HEPA) filter of H10 (HEPA 10), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10¹⁶, a passage number of particles in size of 0.2 µm less than or equal to 237×10¹³, a passage number of particles in size of 0.3 µm less than or equal to 102×10¹³, a passage number of particles in size of 0.5 µm size less than or equal to 352×10¹², a passage number of particles in size of 1 µm less than or equal to 832×10¹¹, a passage number of particles in size of 2.5 µm size less than or equal to 124×10¹¹, a passage number of particles in size of 5 µm size less than or equal to 293×10¹⁰, and a passage number of particles in size of 10 µm size less than or equal to 70×10¹⁰, thereby achieving the cleanliness of cleanroom class 16 (CLASS 16).

In a specific embodiment, the fan 21 has a clean air delivery rate (CADR) less than CADR 600, the filter element 22 includes a high efficiency particulate air (HEPA) filter of H10 (HEPA 10), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10¹⁷, a passage number of particles in size of 0.2 µm less than or equal to 237×10¹⁴, a passage number of particles in size of 0.3 µm less than or equal to 102×10¹⁴, a passage number of particles in size of 0.5 µm size less than or equal to 352×10¹³, a passage number of particles in size of 1 µm less than or equal to 832×10¹², a passage number of particles in size of 2.5 µm size less than or equal to 124×10¹², a passage number of particles in size of 5 µm size less than or equal to 293×10¹¹, and a passage number of particles in size of 10 µm size less than or equal to 70×10¹¹, thereby achieving the cleanliness of cleanroom class 17 (CLASS 17).

In a specific embodiment, the fan 21 has a clean air delivery rate (CADR) less than CADR 600, the filter element 22 includes a high efficiency particulate air (HEPA) filter of H11 (HEPA 11), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10¹⁸, a passage number of particles in size of 0.2 µm less than or equal to 237×10¹⁵, a passage number of particles in size of 0.3 µm less than or equal to 102×10¹⁵, a passage number of particles in size of 0.5 µm size less than or equal to 352×10¹⁴, a passage number of particles in size of 1 µm less than or equal to 832×10¹³, a passage number of particles in size of 2.5 µm size less than or equal to 124×10¹³, a passage number of particles in size of 5 µm size less than or equal to 293×10¹², and a passage number of particles in size of 10 µm size less than or equal to 70×10¹², thereby achieving the cleanliness of cleanroom class 18 (CLASS 18).

In a specific embodiment, the fan 21 has a clean air delivery rate (CADR) less than CADR 600, the filter element 22 includes a high efficiency particulate air (HEPA) filter of H11 (HEPA 11), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10¹⁹, a passage number of particles in size of 0.2 µm less than or equal to 237×10¹⁶, a passage number of particles in size of 0.3 µm less than or equal to 102×10¹⁶, a passage number of particles in size of 0.5 µm size less than or equal to 352×10¹⁵, a passage number of particles in size of 1 µm less than or equal to 832×10¹⁴, a passage number of particles in size of 2.5 µm size less than or equal to 124×10¹⁴, a passage number of particles in size of 5 µm size less than or equal to 293×10¹³, and a passage number of particles in size of 10 µm size less than or equal to 70×10¹³, thereby achieving the cleanliness of cleanroom class 19 (CLASS 19).

In a specific embodiment, the fan 21 has a clean air delivery rate (CADR) less than CADR 600, the filter element 22 includes a high efficiency particulate air (HEPA) filter of H11 (HEPA 11), and the fan 21 of the cleaning device 2 is enabled to generate the directional circular airflow in the indoor field B for rapidly guiding the air pollution to pass through the filter element 22 multiple times for filtration to a level close to zero and through the sterilization component 23 for sterilization, so that the gas state in the indoor field B is formed to have one selected from the group consisting of a passage number of particles in size of 0.1 µm less than or equal to 1×10²⁰, a passage number of particles in size of 0.2 µm less than or equal to 237×10¹⁷, a passage number of particles in size of 0.3 µm less than or equal to 102×10¹⁷, a passage number of particles in size of 0.5 µm size less than or equal to 352×10¹⁶, a passage number of particles in size of 1 µm less than or equal to 832×10¹⁵, a passage number of particles in size of 2.5 µm size less than or equal to 124×10¹⁵, a passage number of particles in size of 5 µm size less than or equal to 293×10¹⁴, and a passage number of particles in size of 10 µm size less than or equal to 70×10¹⁴, thereby achieving the cleanliness of cleanroom class 20 (CLASS 20).

In summary, the present disclosure provides an indoor air cleaning system. By disposing a plurality of gas detectors and at least one cleaning device, the gas detectors can monitor, determine and output the air pollution information of outdoor and indoor at any time. Then, the cloud computing server receives the air pollution information via IOT communication, stores the air pollution information to an air pollution big data database, performs an intelligence computing to compare and determine the location of the air pollution position, and issues a control command to the fan of the cleaning device via IOT communication for adjusting the enabling cycle and air volume, so that a directional circular airflow is generated in the indoor field, and the air pollution is rapidly guided to pass through the filter element multiple times for filtration to a level close to zero and pass through the sterilization component for sterilization, thereby improving the purification efficiency and reducing the environmental noises in the indoor field. Such indoor air cleaning system forms the detecting-cleaning prevention effectiveness of locating the air pollution, guiding the air pollution, and purifying the air pollution to a level close to zero in the indoor field. Moreover, the filter element of the cleaning device employs the HEPA filter screen to filter the air pollution, and the fan of the cleaning device employs a specific CADR (Clean Air Delivery Rate) to generate the directional circular airflow, so that the gas state of the indoor field can reach the cleanroom class by a cleanliness specification based on the passage number of particles, and the impact and injury for human health caused by the gas hazards in the environment can be avoided. The present disclosure is extremely industrially applicable.

## Claims

1. An indoor air cleaning system, **characterized by** comprising:
a plurality of gas detectors (1) disposed in an outdoor field (A) and an indoor field (B) for detecting air pollution information and outputting thereof via Internet of Things (IOT) communication;
at least one cleaning device (2) comprising a fan (21), a filter element (22) and a sterilization component (23), wherein the fan (21) is enabled and controlled via IOT communication to guide an air pollution to pass through the filter element (22) for filtration, and pass through the sterilization component (23) for sterilization, and wherein the fan (21) has a specific clean air delivery rate (CADR) for generating a directional circular airflow, and the filter element (22) comprises a high efficiency particulate air (HEPA) filter screen; and
a cloud computing server (4) receiving the air pollution information of the outdoor field (A) and the indoor field (B) via IOT communication, storing the air pollution information to an air pollution big data database, performing an intelligent computing for comparison based on the air pollution big data database, and intelligently selecting and issuing a control command via IOT communication to enable the fan (21) of the at least one cleaning device (2) to generate the directional circular airflow for rapidly guiding the air pollution to pass through the filter element (22) multiple times for filtration to a level close to zero and pass through the sterilization component (23) for sterilization, thereby reaching a gas state in the indoor field (B) to a cleanroom class by a cleanliness specification based on a passage number of particles.

2. The indoor air cleaning system according to claim 1, wherein the air pollution is at least one selected from the group consisting of particulate matter, carbon monoxide, carbon dioxide, ozone, sulfur dioxide, nitrogen dioxide, lead, total volatile organic compounds (TVOC), formaldehyde, bacteria, fungi, virus and a combination thereof, and wherein the intelligent computing comprises an artificial intelligence (AI) computing and an edge computing.

3. The indoor air cleaning system according to claim 1, wherein the indoor field (B) is disposed with a plurality of gas detectors (1) and a plurality of cleaning devices (2), and wherein at least two of the plurality of gas detectors (1) detect and output the air pollution information to the cloud computing server (4) via IOT communication for locating an air pollution position and intelligently issuing the control command to the plurality of cleaning devices (2), thereby enabling the fan (21) of one of the plurality of cleaning devices (2) which is near the air pollution position to generate a directional airflow and then enabling the fans (21) of others of the plurality of cleaning devices (2), so that the directional circular airflow is generated to rapidly guide the air pollution to pass through the filter element (22) multiple times for filtration to a level close to zero and through the sterilization component (23) for sterilization, and the gas state in the indoor field (B) reaches the cleanroom class by the cleanliness specification based on the passage number of particles.

4. The indoor air cleaning system according to claim 1, wherein the fan (21) of the at least one cleaning device (2) is enabled according to the air pollution information detected in the indoor field (B), and through the intelligent computing by the cloud computing server (4) for comparison based on the big data database, an enabling cycle and an air volume of the fan (21) of the at least one cleaning device (2) is dynamically adjusted in real time for generating the directional circular airflow, so that the air pollution is rapidly guided to pass through the filter element (22) multiple times for filtration to a level close to zero and through the sterilization component (23) for sterilization, a cleaning efficiency of the indoor field (B) is improved and an environmental noises of the indoor field (B) is reduced, and the gas state in the indoor field (B) reaches the cleanroom class by the cleanliness specification based on the passage number of particles.

5. The indoor air cleaning system according to claim 1, wherein the fan (21) of the at least one cleaning device (2) is enabled to generate the directional circular airflow in the indoor field (B) for rapidly guiding the air pollution to pass through the filter element (22) multiple times for filtration to a level close to zero and through the sterilization component (23) for sterilization, and wherein operation conditions of the fan (21) and the cleanliness specification of the indoor field (B) are one selected from the group consisting of:
(1) a clean air delivery rate (CADR) of the fan (21) is greater than CADR 1500, the filter element (22) comprises a nanometer filter, and the gas state in the indoor field (B) is formed to have one selected from the group consisting of a passage number of particles in size of 0.1 µm less than or equal to 1×10², and a passage number of particles in size of 0.2 µm less than or equal to 2, thereby achieving the cleanliness of cleanroom class 1 (CLASS 1); and
(2) a clean air delivery rate (CADR) of the fan (21) is greater than CADR 1500, the filter element (22) comprises an ultra low particulate air (ULPA) filter of U17 (ULPA 17), and the gas state in the indoor field (B) is formed to have one selected from the group consisting of a passage number of particles in size of 0.1 µm is less than or equal to 1×10², a passage number of particles in size of 0.2 µm less than or equal to 24, a passage number of particles in size of 0.3 µm less than or equal to 10, and a passage number of particles in size of 0.5 µm size less than or equal to 4, thereby achieving the cleanliness of cleanroom class 2 (CLASS 2).

6. The indoor air cleaning system according to claim 1, wherein the fan (21) of the at least one cleaning device (2) is enabled to generate the directional circular airflow in the indoor field (B) for rapidly guiding the air pollution to pass through the filter element (22) multiple times for filtration to a level close to zero and through the sterilization component (23) for sterilization, and wherein operation conditions of the fan (21) and the cleanliness specification of the indoor field (B) are one selected from the group consisting of:
(1) a clean air delivery rate (CADR) of the fan (21) is greater than CADR 1000, the filter element (22) comprises an ultra low particulate air (ULPA) filter of U16 (ULPA 16), and the gas state in the indoor field (B) is formed to have one selected from the group consisting of a passage number of particles in size of 0.1 µm less than or equal to 1×10³, a passage number of particles in size of 0.2 µm less than or equal to 237, a passage number of particles in size of 0.3 µm less than or equal to 102, a passage number of particles in size of 0.5 µm size less than or equal to 35, and a passage number of particles in size of 1 µm less than or equal to 8, thereby achieving the cleanliness of cleanroom class 3 (CLASS 3); and
(2) a clean air delivery rate (CADR) of the fan (21) is greater than CADR 1000, the filter element (22) comprises an ultra low particulate air (ULPA) filter of U15 (ULPA 15), and the gas state in the indoor field (B) is formed to have one selected from the group consisting of a passage number of particles in size of 0.1 µm less than or equal to 1×10⁴, a passage number of particles in size of 0.2 µm less than or equal to 237×10¹, a passage number of particles in size of 0.3 µm less than or equal to 102×10¹, a passage number of particles in size of 0.5 µm size less than or equal to 352, a passage number of particles in size of 1 µm less than or equal to 83, and a passage number of particles in size of 2.5 µm size less than or equal to 12, thereby achieving the cleanliness of cleanroom class 4 (CLASS 4).

7. The indoor air cleaning system according to claim 1, wherein the fan (21) of the at least one cleaning device (2) is enabled to generate the directional circular airflow in the indoor field (B) for rapidly guiding the air pollution to pass through the filter element (22) multiple times for filtration to a level close to zero and through the sterilization component (23) for sterilization, and wherein operation conditions of the fan (21) and the cleanliness specification of the indoor field (B) are one selected from the group consisting of:
(1) a clean air delivery rate (CADR) of the fan (21) is greater than CADR 1000, the filter element (22) comprises an ultra low particulate air (ULPA) filter of U15 (ULPA 15), and the gas state in the indoor field (B) is formed to have one selected from the group consisting of a passage number of particles in size of 0.1 µm less than or equal to 1×10⁵, a passage number of particles in size of 0.2 µm less than or equal to 237×10², a passage number of particles in size of 0.3 µm less than or equal to 102×10², a passage number of particles in size of 0.5 µm size less than or equal to 352×10¹, a passage number of particles in size of 1 µm less than or equal to 832, a passage number of particles in size of 2.5 µm size less than or equal to 124, and a passage number of particles in size of 5 µm size less than or equal to 29, thereby achieving the cleanliness of cleanroom class 5 (CLASS 5); and
(2) a clean air delivery rate (CADR) of the fan (21) is greater than CADR 600, the filter element (22) comprises a high efficiency particulate air (HEPA) filter of H14 (HEPA 14), and the gas state in the indoor field (B) is formed to have one selected from the group consisting of a passage number of particles in size of 0.1 µm less than or equal to 1×10⁶, a passage number of particles in size of 0.2 µm less than or equal to 237×10³, a passage number of particles in size of 0.3 µm less than or equal to 102×10³, a passage number of particles in size of 0.5 µm size less than or equal to 352×10², a passage number of particles in size of 1 µm less than or equal to 832×10¹, a passage number of particles in size of 2.5 µm size less than or equal to 124×10¹, a passage number of particles in size of 5 µm size less than or equal to 293, and a passage number of particles in size of 10 µm size less than or equal to 70, thereby achieving the cleanliness of cleanroom class 6 (CLASS 6).

8. The indoor air cleaning system according to claim 1, wherein the fan (21) of the at least one cleaning device (2) is enabled to generate the directional circular airflow in the indoor field (B) for rapidly guiding the air pollution to pass through the filter element (22) multiple times for filtration to a level close to zero and through the sterilization component (23) for sterilization, and wherein operation conditions of the fan (21) and the cleanliness specification of the indoor field (B) are one selected from the group consisting of:
(1) a clean air delivery rate (CADR) of the fan (21) is greater than CADR 600, the filter element (22) comprises a high efficiency particulate air (HEPA) filter of H13 (HEPA 13), and the gas state in the indoor field (B) is formed to have one selected from the group consisting of a passage number of particles in size of 0.1 µm less than or equal to 1×10⁷, a passage number of particles in size of 0.2 µm less than or equal to 237×10⁴, a passage number of particles in size of 0.3 µm less than or equal to 102×10⁴, a passage number of particles in size of 0.5 µm size less than or equal to 352×10³, a passage number of particles in size of 1 µm less than or equal to 832×10², a passage number of particles in size of 2.5 µm size less than or equal to 124×10², a passage number of particles in size of 5 µm size less than or equal to 293×10¹, and a passage number of particles in size of 10 µm size less than or equal to 70×10¹, thereby achieving the cleanliness of cleanroom class 7 (CLASS 7); and
(2) a clean air delivery rate (CADR) of the fan (21) is greater than CADR 600, the filter element (22) comprises high efficiency particulate air (HEPA) filter of H13 (HEPA 13), and the gas state in the indoor field (B) is formed to have one selected from the group consisting of a passage number of particles in size of 0.1 µm less than or equal to 1×10⁸, a passage number of particles in size of 0.2 µm less than or equal to 237×10⁵, a passage number of particles in size of 0.3 µm less than or equal to 102×10⁵, a passage number of particles in size of 0.5 µm size less than or equal to 352×10⁴, a passage number of particles in size of 1 µm less than or equal to 832×10³, a passage number of particles in size of 2.5 µm size less than or equal to 124×10³, a passage number of particles in size of 5 µm size less than or equal to 293×10², and a passage number of particles in size of 10 µm size less than or equal to 70×10², thereby achieving the cleanliness of cleanroom class 8 (CLASS 8).

9. The indoor air cleaning system according to claim 1, wherein the fan (21) of the at least one cleaning device (2) is enabled to generate the directional circular airflow in the indoor field (B) for rapidly guiding the air pollution to pass through the filter element (22) multiple times for filtration to a level close to zero and through the sterilization component (23) for sterilization, and wherein operation conditions of the fan (21) and the cleanliness specification of the indoor field (B) are one selected from the group consisting of:
(1) a clean air delivery rate (CADR) of the fan (21) is greater than CADR 600, the filter element (22) comprises a high efficiency particulate air (HEPA) filter of H12 (HEPA 12), and the gas state in the indoor field (B) is formed to have one selected from the group consisting of a passage number of particles in size of 0.1 µm less than or equal to 1×10⁹, a passage number of particles in size of 0.2 µm less than or equal to 237×10⁶, a passage number of particles in size of 0.3 µm less than or equal to 102×10⁶, a passage number of particles in size of 0.5 µm size less than or equal to 352×10⁵, a passage number of particles in size of 1 µm less than or equal to 832×10², a passage number of particles in size of 2.5 µm size less than or equal to 124×10⁴, a passage number of particles in size of 5 µm size less than or equal to 293×10³, and a passage number of particles in size of 10 µm size less than or equal to 70×10³, thereby achieving the cleanliness of cleanroom class 9 (CLASS 9); and
(2) a clean air delivery rate (CADR) of the fan (21) is less than CADR 600, the filter element (22) comprises high efficiency particulate air (HEPA) filter of H12 (HEPA 12), and the gas state in the indoor field (B) is formed to have one selected from the group consisting of a passage number of particles in size of 0.1 µm less than or equal to 1×10¹⁰, a passage number of particles in size of 0.2 µm less than or equal to 237×10⁷, a passage number of particles in size of 0.3 µm less than or equal to 102×10⁷, a passage number of particles in size of 0.5 µm size less than or equal to 352×10⁶, a passage number of particles in size of 1 µm less than or equal to 832×10⁵, a passage number of particles in size of 2.5 µm size less than or equal to 124×10⁵, a passage number of particles in size of 5 µm size less than or equal to 293×10⁴, and a passage number of particles in size of 10 µm size less than or equal to 70×10⁴, thereby achieving the cleanliness of cleanroom class 10 (CLASS 10).

10. The indoor air cleaning system according to claim 1, wherein the fan (21) of the at least one cleaning device (2) is enabled to generate the directional circular airflow in the indoor field (B) for rapidly guiding the air pollution to pass through the filter element (22) multiple times for filtration to a level close to zero and through the sterilization component (23) for sterilization, and wherein operation conditions of the fan (21) and the cleanliness specification of the indoor field (B) are one selected from the group consisting of:
(1) a clean air delivery rate (CADR) of the fan (21) is less than CADR 600, the filter element (22) comprises a high efficiency particulate air (HEPA) filter of H12 (HEPA 12), and the gas state in the indoor field (B) is formed to have one selected from the group consisting of a passage number of particles in size of 0.1 µm less than or equal to 1×10¹¹, a passage number of particles in size of 0.2 µm less than or equal to 237×10⁸, a passage number of particles in size of 0.3 µm less than or equal to 102×10⁸, a passage number of particles in size of 0.5 µm size less than or equal to 352×10⁷, a passage number of particles in size of 1 µm less than or equal to 832×10⁶, a passage number of particles in size of 2.5 µm size less than or equal to 124×10⁶, a passage number of particles in size of 5 µm size less than or equal to 293×10⁵, and a passage number of particles in size of 10 µm size less than or equal to 70×10⁵, thereby achieving the cleanliness of cleanroom class 11 (CLASS 11); and
(2) a clean air delivery rate (CADR) of the fan (21) is less than CADR 600, the filter element (22) comprises high efficiency particulate air (HEPA) filter of H12 (HEPA 12), and the gas state in the indoor field (B) is formed to have one selected from the group consisting of a passage number of particles in size of 0.1 µm less than or equal to 1×10¹², a passage number of particles in size of 0.2 µm less than or equal to 237×10⁹, a passage number of particles in size of 0.3 µm less than or equal to 102×10⁹, a passage number of particles in size of 0.5 µm size less than or equal to 352×10⁸, a passage number of particles in size of 1 µm less than or equal to 832×10⁷, a passage number of particles in size of 2.5 µm size less than or equal to 124×10⁷, a passage number of particles in size of 5 µm size less than or equal to 293×10⁶, and a passage number of particles in size of 10 µm size less than or equal to 70×10⁶, thereby achieving the cleanliness of cleanroom class 12 (CLASS 12).

11. The indoor air cleaning system according to claim 1, wherein the fan (21) of the at least one cleaning device (2) is enabled to generate the directional circular airflow in the indoor field (B) for rapidly guiding the air pollution to pass through the filter element (22) multiple times for filtration to a level close to zero and through the sterilization component (23) for sterilization, and wherein operation conditions of the fan (21) and the cleanliness specification of the indoor field (B) are one selected from the group consisting of:
(1) a clean air delivery rate (CADR) of the fan (21) is less than CADR 600, the filter element (22) comprises a high efficiency particulate air (HEPA) filter of H12 (HEPA 12), and the gas state in the indoor field (B) is formed to have one selected from the group consisting of a passage number of particles in size of 0.1 µm less than or equal to 1×10¹³, a passage number of particles in size of 0.2 µm less than or equal to 237×10¹⁰, a passage number of particles in size of 0.3 µm less than or equal to 102×10¹⁰, a passage number of particles in size of 0.5 µm size less than or equal to 352×10⁹, a passage number of particles in size of 1 µm less than or equal to 832×10⁸, a passage number of particles in size of 2.5 µm size less than or equal to 124×10⁸, a passage number of particles in size of 5 µm size less than or equal to 293×10⁷, and a passage number of particles in size of 10 µm size less than or equal to 70×10⁷, thereby achieving the cleanliness of cleanroom class 13 (CLASS 13); and
(2) a clean air delivery rate (CADR) of the fan (21) is less than CADR 600, the filter element (22) comprises high efficiency particulate air (HEPA) filter of H12 (HEPA 12), and the gas state in the indoor field (B) is formed to have one selected from the group consisting of a passage number of particles in size of 0.1 µm less than or equal to 1×10¹⁴, a passage number of particles in size of 0.2 µm less than or equal to 237×10¹¹, a passage number of particles in size of 0.3 µm less than or equal to 102×10¹¹, a passage number of particles in size of 0.5 µm size less than or equal to 352×10¹⁰, a passage number of particles in size of 1 µm less than or equal to 832×10⁹, a passage number of particles in size of 2.5 µm size less than or equal to 124×10⁹, a passage number of particles in size of 5 µm size less than or equal to 293×10⁸, and a passage number of particles in size of 10 µm size less than or equal to 70×10⁸, thereby achieving the cleanliness of cleanroom class 14 (CLASS 14).

12. The indoor air cleaning system according to claim 1, wherein the fan (21) of the at least one cleaning device (2) is enabled to generate the directional circular airflow in the indoor field (B) for rapidly guiding the air pollution to pass through the filter element (22) multiple times for filtration to a level close to zero and through the sterilization component (23) for sterilization, and wherein operation conditions of the fan (21) and the cleanliness specification of the indoor field (B) are one selected from the group consisting of:
(1) a clean air delivery rate (CADR) of the fan (21) is less than CADR 600, the filter element (22) comprises a high efficiency particulate air (HEPA) filter of H12 (HEPA 12), and the gas state in the indoor field (B) is formed to have one selected from the group consisting of a passage number of particles in size of 0.1 µm less than or equal to 1×10¹⁵, a passage number of particles in size of 0.2 µm less than or equal to 237×10¹², a passage number of particles in size of 0.3 µm less than or equal to 102×10¹², a passage number of particles in size of 0.5 µm size less than or equal to 352×10¹¹, a passage number of particles in size of 1 µm less than or equal to 832×10¹⁰, a passage number of particles in size of 2.5 µm size less than or equal to 124×10¹⁰, a passage number of particles in size of 5 µm size less than or equal to 293×10⁹, and a passage number of particles in size of 10 µm size less than or equal to 70×10⁹, thereby achieving the cleanliness of cleanroom class 15 (CLASS 15); and
(2) a clean air delivery rate (CADR) of the fan (21) is less than CADR 600, the filter element (22) comprises high efficiency particulate air (HEPA) filter of H10 (HEPA 10), and the gas state in the indoor field (B) is formed to have one selected from the group consisting of a passage number of particles in size of 0.1 µm less than or equal to 1×10¹⁶, a passage number of particles in size of 0.2 µm less than or equal to 237×10¹³, a passage number of particles in size of 0.3 µm less than or equal to 102×10¹³, a passage number of particles in size of 0.5 µm size less than or equal to 352×10¹², a passage number of particles in size of 1 µm less than or equal to 832×10¹¹, a passage number of particles in size of 2.5 µm size less than or equal to 124×10¹¹, a passage number of particles in size of 5 µm size less than or equal to 293×10¹⁰, and a passage number of particles in size of 10 µm size less than or equal to 70×10¹⁰, thereby achieving the cleanliness of cleanroom class 16 (CLASS 16).

13. The indoor air cleaning system according to claim 1, wherein the fan (21) of the at least one cleaning device (2) is enabled to generate the directional circular airflow in the indoor field (B) for rapidly guiding the air pollution to pass through the filter element (22) multiple times for filtration to a level close to zero and through the sterilization component (23) for sterilization, and wherein operation conditions of the fan (21) and the cleanliness specification of the indoor field (B) are one selected from the group consisting of:
(1) a clean air delivery rate (CADR) of the fan (21) is less than CADR 600, the filter element (22) comprises a high efficiency particulate air (HEPA) filter of H12 (HEPA 12), and the gas state in the indoor field (B) is formed to have one selected from the group consisting of a passage number of particles in size of 0.1 µm less than or equal to 1×10¹⁷, a passage number of particles in size of 0.2 µm less than or equal to 237×10¹⁴, a passage number of particles in size of 0.3 µm less than or equal to 102×10¹⁴, a passage number of particles in size of 0.5 µm size less than or equal to 352×10¹³, a passage number of particles in size of 1 µm less than or equal to 832×10¹², a passage number of particles in size of 2.5 µm size less than or equal to 124×10¹², a passage number of particles in size of 5 µm size less than or equal to 293×10¹¹, and a passage number of particles in size of 10 µm size less than or equal to 70×10¹¹, thereby achieving the cleanliness of cleanroom class 17 (CLASS 17); and
(2) a clean air delivery rate (CADR) of the fan (21) is less than CADR 600, the filter element (22) comprises high efficiency particulate air (HEPA) filter of H11 (HEPA 11), and the gas state in the indoor field (B) is formed to have one selected from the group consisting of a passage number of particles in size of 0.1 µm less than or equal to 1×10¹⁸, a passage number of particles in size of 0.2 µm less than or equal to 237×10¹⁵, a passage number of particles in size of 0.3 µm less than or equal to 102×10¹⁵, a passage number of particles in size of 0.5 µm size less than or equal to 352×10¹⁴, a passage number of particles in size of 1 µm less than or equal to 832×10¹³, a passage number of particles in size of 2.5 µm size less than or equal to 124×10¹³, a passage number of particles in size of 5 µm size less than or equal to 293×10¹², and a passage number of particles in size of 10 µm size less than or equal to 70×10¹², thereby achieving the cleanliness of cleanroom class 18 (CLASS 18).

14. The indoor air cleaning system according to claim 1, wherein the fan (21) of the at least one cleaning device (2) is enabled to generate the directional circular airflow in the indoor field (B) for rapidly guiding the air pollution to pass through the filter element (22) multiple times for filtration to a level close to zero and through the sterilization component (23) for sterilization, and wherein operation conditions of the fan (21) and the cleanliness specification of the indoor field (B) are one selected from the group consisting of:
(1) a clean air delivery rate (CADR) of the fan (21) is less than CADR 600, the filter element (22) comprises a high efficiency particulate air (HEPA) filter of H11 (HEPA 11), and the gas state in the indoor field (B) is formed to have one selected from the group consisting of a passage number of particles in size of 0.1 µm less than or equal to 1×10¹⁹, a passage number of particles in size of 0.2 µm less than or equal to 237×10¹⁶, a passage number of particles in size of 0.3 µm less than or equal to 102×10¹⁶, a passage number of particles in size of 0.5 µm size less than or equal to 352×10¹⁵, a passage number of particles in size of 1 µm less than or equal to 832×10¹⁴, a passage number of particles in size of 2.5 µm size less than or equal to 124×10¹⁴, a passage number of particles in size of 5 µm size less than or equal to 293×10¹³, and a passage number of particles in size of 10 µm size less than or equal to 70×10¹³, thereby achieving the cleanliness of cleanroom class 19 (CLASS 19); and
(2) a clean air delivery rate (CADR) of the fan (21) is less than CADR 600, the filter element (22) comprises high efficiency particulate air (HEPA) filter of H11 (HEPA 11), and the gas state in the indoor field (B) is formed to have one selected from the group consisting of a passage number of particles in size of 0.1 µm less than or equal to 1×10²⁰, a passage number of particles in size of 0.2 µm less than or equal to 237×10¹⁷, a passage number of particles in size of 0.3 µm less than or equal to 102×10¹⁷, a passage number of particles in size of 0.5 µm size less than or equal to 352×10¹⁶, a passage number of particles in size of 1 µm less than or equal to 832×10¹⁵, a passage number of particles in size of 2.5 µm size less than or equal to 124×10¹⁵, a passage number of particles in size of 5 µm size less than or equal to 293×10¹⁴, and a passage number of particles in size of 10 µm size less than or equal to 70×10¹⁴, thereby achieving the cleanliness of cleanroom class 20 (CLASS 20).

15. The indoor air cleaning system according to claim 1, wherein the cloud computing server (4) comprises a wireless network cloud computing service module (41), a cloud control service unit (42), a device management unit (43) and an application program unit (44), and each of the plurality of gas detectors (1) comprises a controlling circuit board (11), a gas detection main part (12), a microprocessor (13) and a communicator (14), wherein the gas detection main part (12), the microprocessor (13) and the communicator (14) are integrally packaged on the controlling circuit board (11) and electrically connected to the controlling circuit board (11), the microprocessor (13) controls the detection of the gas detection main part (12), the gas detection main part (12) detects the air pollution, and the microprocessor (13) processes and provides the air pollution information to the communicator (14) for external transmission, and wherein at least one gas exchanging device (3) is disposed in the indoor field (B) for guiding the air from the outdoor field (A) to the indoor field (B) so as to achieve a gas exchanging, wherein through the cloud computing server (4) intelligently compares the air pollution information between the outdoor field (A) and the indoor field (B), when the air pollution information of the indoor field (B) is greater than the air pollution information of the outdoor field (A), the cloud computing server (4) issues a control command via IOT communication to enable the gas exchanging device (3) to guide the air from the outdoor field (A) to the indoor field (B) for gas exchanging.
